# EUROPEAN PATENT APPLICATION

(11) **EP 4 799 662 A1**
(43) Date of publication of application: **02.09.2026**
(21) Application number: 24881009.5
(22) Date of filing: 27.05.2024
(51) Int. Cl.: A61M 5/142, G16H 20/60

(54) **POSTPRANDIAL INSULIN INFUSION SYSTEM AND METHOD BASED ON RECOGNIZABLE FEATURES OF DIABETIC PATIENT**

(30) Priority: 25.10.2023 WO PCT/CN2023/126418
(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2024/095393
(87) International publication number: WO 2025/086630

(57) **Abstract**

The invention discloses a method and system for postprandial insulin infusion based on identifiable feature of diabetic, acquiring a food image by an imaging mechanism, and analyzing the food image by the food image recognition model to obtain food data, retrieving a narrow insulin algorithm adapted to the patient's own identifiable features from the cluster of narrow insulin algorithms according to the identifiable features of the patient, for calculation of insulin infusion amount during meals, compared to the preset insulin algorithm, the narrow insulin algorithm is more applicable to the physiological characteristics and living habit of the patient, the calculated postprandial insulin infusion amount is more accurate, and is beneficial for the treatment of diabetic.

## Description

### TECHNICAL FIELD

The invention mainly relates to the field of medical device, and in particular to a method and system for postprandial insulin infusion based on identifiable feature of diabetic.

### BACKGROUND

The pancreas of healthy people can automatically secrete the required insulin/glucagon according to the glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods of continuously monitoring blood glucose are based on in vivo glucose monitoring devices, which use disposable transdermal sensors inserted into the skin to measure glucose concentrations in interstitial fluids and send the blood glucose data through a transmitter to the remote device in real-time for the user to view. This monitoring method is called Continuous Glucose Monitoring (CGM). Based on the blood glucose value detected by the CGM, the insulin pump automatically adjusts the amount of insulin required by the patient and infuses it subcutaneously, thus constituting a closed-loop artificial pancreas.

When a patient infuses insulin during a meal period, the effect of food on the patient's blood glucose needs to be considered, and different food types and weights have different effects on the patient's blood glucose, which can make accurate infusion of insulin difficult. The current prior art does not have a suitable method to accurately estimate blood glucose changes as well as insulin infusion amounts from food.

Accordingly, there is a need in the art for a method and system that accurately estimates blood glucose changes and insulin infusion amounts from food during a meal period.

### BRIEF SUMMARY OF THE INVENTION

Embodiments of the invention disclose a method and system for postprandial insulin infusion based on identifiable feature of diabetic, acquiring a food image by an imaging mechanism, and analyzing the food image by the food image recognition model to obtain food data, retrieving a narrow insulin algorithm adapted to the patient's own identifiable features from the cluster of narrow insulin algorithms according to the identifiable features of the patient, for calculation of insulin infusion amount during meals, compared to the preset insulin algorithm, the narrow insulin algorithm is more applicable to the physiological characteristics and living habit of the patient, the calculated postprandial insulin infusion amount is more accurate, and is beneficial for the treatment of diabetic.

The invention discloses a closed-loop artificial pancreas system, comprising an imaging mechanism for acquiring food images on food before a meal; a food image recognition model for recognizing a food image to acquire food data, the food data including at least type and weight of food; a cluster of narrow insulin algorithms assembled by narrow insulin algorithms, in the cluster of narrow insulin algorithm, the narrow insulin algorithms are classified into groups with identifiable features, with common identifiable features within the groups, the patient retrieves the narrow insulin algorithm based on the identifiable features, and determines a postprandial insulin infusion amount of the patient using the narrow insulin algorithm based on the food data; and a closed-loop artificial pancreas for completing the infusion of postprandial insulin and detecting actual blood glucose of the patient, obtaining actual blood glucose data.

According to one aspect of the invention, the groups are independent of each other.

According to one aspect of the invention, the two groups having at least one same identifiable feature are subsets of each other.

According to one aspect of the invention, further comprising a cloud server, the cloud server establishing communication with the imaging mechanism or the closed-loop artificial pancreas.

According to one aspect of the invention, further comprising a food nutrition library, the food data further comprising the content of nutrients of the food, the food nutrition library being retrieved based on the type and weight of the food to determine the content of nutrients of the food.

According to one aspect of the invention, the narrow insulin algorithm determines the postprandial insulin infusion amount based on the content of nutrients.

According to one aspect of the invention, the cluster of narrow insulin algorithms is located in the imaging mechanism, the closed-loop artificial pancreas or the cloud server.

According to one aspect of the invention, the image recognition model is located in the imaging mechanism, the closed-loop artificial pancreas or the cloud server.

According to one aspect of the invention, the food nutrition library is located in the imaging mechanism, the closed-loop artificial pancreas or the cloud server.

According to one aspect of the invention, the identifiable features are associated with at least a physiological state or living habit of the patient, and identifiable features may be added, modified or deleted.

According to one aspect of the invention, the imaging mechanism is a closed-loop artificial pancreas independent image acquisition device.

According to one aspect of the invention, the imaging mechanism is a sub-module of the closed-loop artificial pancreas.

According to one aspect of the invention, the narrow insulin algorithm is also used to simulate the postprandial blood glucose of the patient, based on the postprandial insulin infusion amount, resulting in simulated blood glucose data.

According to one aspect of the invention, the actual blood glucose data and the simulated blood glucose data are compared, and the parameters of the narrow insulin algorithm and/or the image recognition model are corrected according to the comparison result.

The invention also discloses a closed-loop insulin infusion method, comprising providing: an imaging mechanism, a food image recognition model, a cluster of narrow insulin algorithm and a closed-loop artificial pancreas, characterized by comprising the steps of:
I. Before a meal, acquiring a food image for the food using an imaging mechanism;
II. Recognizing the food image by the food image recognition model, acquiring food data related to the food;
III. Retrieving a narrow insulin algorithm according to the identifiable characteristics of the patient from the cluster of insulin algorithms, determining a postprandial insulin infusion amount of the patient by the insulin algorithm based on the food data;
IV. Completing the postprandial insulin infusion by the closed-loop artificial pancreas and detecting, recording actual blood glucose data;

According to one aspect of the invention, in the step II, further comprising a step of confirming the food data by the patient, and if the food data is confirmed by the patient, proceeding to the step III, and vice versa, returning to the step I, or re-recognizing the food image by the food image recognition model.

According to one aspect of the invention, in the step II, the food data acquired include at least the type and weight of the food.

According to one aspect of the invention, further comprising providing a food nutrient library, retrieving the food nutrient library to determine the nutritional content of the food based on the type and weight of the food.

According to one aspect of the invention, in the step III, the postprandial insulin infusion amount is determined by the narrow insulin algorithm based on the nutritional content of the food.

According to one aspect of the invention, further comprising providing a cloud server in which the image recognition model, the cluster of narrow insulin algorithms or the food nutrition library is stored.

According to one aspect of the invention, in the step II, further comprising uploading the food image to the cloud server, and completing food image recognition in the cloud server.

According to one aspect of the invention, in the step III, further comprising uploading the food data to the cloud server, and completing the calculation of the postprandial insulin infusion amount in the cloud server.

According to one aspect of the invention, in the step III, further comprising determining the postprandial insulin infusion amount by the narrow insulin algorithm based on the type and weight of the food.

According to one aspect of the invention, further comprising simulating a postprandial blood glucose of the patient based on the postprandial insulin infusion amount, and obtaining the simulated blood glucose data.

According to one aspect of the invention, further comprising step:
V. Comparing actual blood glucose data and simulated blood glucose data, the parameters of the narrow insulin algorithm are corrected based on the comparison results.

According to one aspect of the invention, the actual blood glucose data coincides in time with the simulated blood glucose data.

According to one aspect of the invention, the narrow insulin algorithm with parameters corrected is assembled into the cluster of narrow insulin algorithm and replaces the narrow insulin algorithm before parameter correction.

Compared with the prior art, the technical solution of the invention has the following advantages:
The invention disclose a method and system for postprandial insulin infusion based on identifiable feature of diabetic, acquiring a food image by an imaging mechanism, and analyzing the food image by the food image recognition model to obtain food data, retrieving a narrow insulin algorithm adapted to the patient's own identifiable features from the cluster of narrow insulin algorithms according to the identifiable features of the patient, for calculation of insulin infusion amount during meals, compared to the preset insulin algorithm, the narrow insulin algorithm is more applicable to the physiological characteristics and living habit of the patient, the calculated postprandial insulin infusion amount is more accurate, and is beneficial for the treatment of diabetic.

Further, determining the patient's postprandial insulin infusion amount based on the type and weight of food can more accurately control the patient's postprandial blood glucose level, and can help the patient's diabetes treatment.

Further, determining the patient's postprandial insulin infusion based on the nutrient content of the food can more accurately control the patient's postprandial blood glucose level and aid in the patient's diabetes treatment.

Further, after the image recognition model recognizes the food data, the postprandial insulin infusion amount needs to be calculated after the patient's confirmation, and the image recognition model is prevented from misjudging or recognition error, the recognition accuracy rate of the image recognition model is improved, and the patient's infusion safety is ensured.

Further, the parameter corrected insulin algorithm can be used by the patient for the next postprandial insulin calculation, with successive iterations of modification of the insulin algorithm parameters, the insulin algorithm will be more and more applicable to the patient, improving the accuracy of the insulin algorithm, enabling more precise control of the postprandial blood glucose level of the patient, facilitating the patient's diabetes treatment.

Further, the parameter corrected narrow insulin algorithms are assembled into an insulin algorithm cluster, and replacing the narrow insulin algorithm before the parameter correction, after every meal, the narrow insulin algorithm can be corrected, its calculation precision and adaptability are gradually improved, and the accuracy of the narrow insulin algorithm is improved, the postprandial blood glucose level of the patient is controlled more precisely, and the diabetes treatment of the patient is helped.

Further, a cloud server can be accessed into the closed-loop artificial pancreas system, by means of the strong computing and storage capacity of the cloud server, it is possible to better serve the recognition of food image, the computation of insulin, the correction of narrow insulin algorithm and the storage and reading of cluster of narrow insulin algorithm, it is possible to optimize the closed-loop artificial pancreas, the computing power of intelligent devices, the problem of insufficient storage capacity.

Further, identifiable features can be added, modified or deleted, due to the large number of diabetic patients, the variety and number of identifiable features are necessarily large, it is not possible to store all identifiable features in a system or server in an exhaustive manner, and the identifiable features can be continuously optimized to meet patient usage as a large number of patients are added, modified or deleted at any time during use.

### BRIEF DESCRIPTION OF THE DRAWINGS

FIG.1 is a schematic diagram of the module relationship of the general closed-loop artificial pancreas insulin infusion control system according to the embodiment of the invention.
FIG.2 is a schematic diagram of the integrated continuous glucose monitoring device according to an embodiment of the invention.
FIG.3 is a schematic diagram of the split continuous glucose monitoring device according to an embodiment of the invention.
FIG.4a is a schematic diagram of the integrated insulin pump according to an embodiment of the invention.
FIG.4b is a schematic diagram of the split insulin pump according to an embodiment of the invention.
FIG. 5a is a schematic diagram of the main interface when the control system is in a first working mode according to an embodiment of the invention.
FIG. 5b is a schematic diagram of the main interface when the control system is in a second working mode according to an embodiment of the invention.
FIG. 6a-FIG. 6c are schematic diagrams of different operations for the control system to turn on the insulin pump function according to embodiments of the invention.
FIG. 7a-FIG. 7c are schematic diagrams of different operations for the control system to turn on the auto mode function according to embodiments of the invention.
FIG. 8a and FIG. 8b show schematic diagrams of the APP interface before and after turning on the auto mode function of the control system according to an embodiment of the invention.
FIG. 9a is a schematic diagram of the interface when the high carbohydrate mode is turned on of the system according to embodiments of the invention.
FIG. 9b is a schematic diagram of the interface when the large meal is selected in the high carbohydrate mode of the system according to embodiments of the invention.
FIG. 9c is a schematic diagram of the interface when the regular meal is selected in the high carbohydrate mode of the system according to embodiments of the invention.
FIG. 9d is a schematic diagram of the interface when the normal carbohydrate mode is turned on of the system according to embodiments of the invention.
FIG. 10 is a schematic diagram of the process of an infusion strategy for pre-infusion and supplemental infusion according to embodiments of the invention.
FIG. 11a is a schematic diagram of a closed-loop infusion system based on food image recognition according to an embodiment of the invention.
FIG. 11b is a schematic diagram of a closed-loop infusion method based on food image recognition according to an embodiment of the invention.
FIG. 11c is a schematic diagram of a closed-loop infusion method based on image recognition of food according to another embodiment of the invention.
FIG. 12 is a schematic diagram of another closed-loop infusion method based on image recognition of food according to an embodiment of the invention.

### DETAILED DESCRIPTION

As described previously, when a patient infuses insulin during a meal period, the effect of food on the patient's blood glucose needs to be considered, and different food types and weights have different effects on the patient's blood glucose, which can make accurate infusion of insulin difficult. The current prior art does not have a suitable method to accurately estimate blood glucose changes as well as insulin infusion amounts from food.

To solve this problem, the invention provides a method and system for postprandial insulin infusion based on identifiable feature of diabetic, acquiring a food image by an imaging mechanism, and analyzing the food image by the food image recognition model to obtain food data, retrieving a narrow insulin algorithm adapted to the patient's own identifiable features from the cluster of narrow insulin algorithms according to the identifiable features of the patient, for calculation of insulin infusion amount during meals, compared to the preset insulin algorithm, the narrow insulin algorithm is more applicable to the physiological characteristics and living habit of the patient, the calculated postprandial insulin infusion amount is more accurate, and is beneficial for the treatment of diabetic.

Various exemplary embodiments of the invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units can be exaggerated relative to other parts.

The following description of the exemplary embodiments is merely illustrative, and is not intended to be in any way limiting the invention and its application or use. The techniques, methods, and devices that are known to those of ordinary skill in the art cannot be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

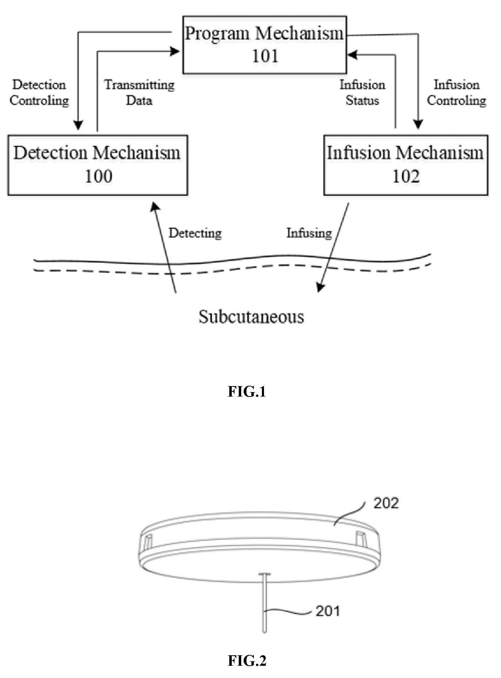

FIG.1 is a schematic diagram of the module relationship of a general closed-loop artificial pancreas insulin infusion control system according to the embodiment of the invention.

The closed-loop artificial pancreas insulin infusion control system disclosed in the embodiment of the invention mainly includes a detection mechanism 100, a program mechanism 101, and an infusion mechanism 102.

The detection mechanism 100 is used to continuously detect the user's real-time blood glucose (BG) level. Generally, detection mechanism 100 is a Continuous Glucose Monitoring (CGM) for detecting real-time BG, monitoring BG changes, and sending them to the program mechanism 101. The CGM includes an implantable sensor, the sensor is connected to a transmitter, the transmitter further includes a memory, a processor, a communication interface, and the like, and the transmitter is used for transmitting at least information about the blood glucose data monitored by the CGM, as well as information about an identifier of the CGM, and the like.

The infusion mechanism 102 includes the essential mechanical assemblies and electronic control unit used to infuse insulin, such as reservoir, drive structures, fluid path and infusion needles, power supplies, circuit boards, and so on, and is controlled by program mechanism 101. Generally, the infusion mechanism 102 is an insulin pump, and the electronic control unit includes a memory, a processor, a communication interface, etc. According to the current insulin infusion dose sent by program mechanism 101, infusion mechanism 102 injects the current insulin dose required into the user's body. At the same time, the real-time infusion status of infusion mechanism 102 can also be fed back to program mechanism 101.

Program mechanism 101 is used to control the detection mechanism 100 and the infusion mechanism 102. Therefore, program mechanism 101 is connected to detection mechanism 100 and infusion mechanism 102, respectively. Here, the connection refers to a conventional electrical connection or a wireless connection.

The embodiment of the invention does not limit the specific positions and connection relationships of the detection mechanism 100, the program mechanism 101 and the infusion mechanism 102, as long as the aforementioned functional conditions can be satisfied.

As in an embodiment of the invention, the three are electrically connected to form a single part. Therefore, the three mechanisms can be attached on only one position of the user's skin. If the three mechanisms are connected as a whole and attached in only one position, the number of the device on the user skin will be reduced, thereby reducing the interference of more attached devices on user activities. At the same time, it also effectively solves the problem of poor wireless communication between separating devices, further enhancing the user experience.

Another embodiment of the invention is that the program mechanism 101 and the infusion mechanism 102 are electrically connected to form a single part, while the detection mechanism 100 is separately provided in another part. At this time, the detection mechanism 100 and the program mechanism 101 transmit wireless signals to realize the mutual connection. Therefore, program mechanism 101 and infusion mechanism 102 can be attached to the user's skin position while the detection mechanism 100 is attached to the other position.

Another embodiment of the invention is that the program mechanism 101 and the detection mechanism 100 are electrically connected, forming a single part, while the infusion mechanism 102 is separately provided in another part. The infusion mechanism 102 and the program mechanism 101 transmit wireless signals to realize the mutual connection. Therefore, program mechanism 101 and the detection mechanism 100 can be attached to the same position of the user's skin while the infusion mechanism 102 is attached to the other position.

Another embodiment of the invention is that the three are provided in different parts, thus being attached to different positions. Simultaneously, program mechanism 101, detection mechanism 100, and infusion mechanism 102 transmit wireless signals to realize the mutual connection.

Another embodiment of the invention is that the three are provided in different parts, the detection mechanism 100 and the infusion mechanism 102 are attached to different locations of the user's skin, respectively, while the program mechanism 101 does not have to be attached to the skin, and control the detection mechanism 100 and the infusion mechanism 102 by means of a handheld or portable device. At this time, the program mechanism 101 transmits wireless signals to the detection mechanism 100 and the infusion mechanism 102, respectively, to achieve connection with each other.

The wireless connections described in the foregoing embodiments include, but are not limited to, radio frequency (RF) communications (e.g., radio frequency identification (RFID), Zigbee communication protocol, WiFi, infrared, wireless Universal Serial Bus (USB), Ultra Wide Band (UWB), Bluetooth^{®} communication protocol, and cellular communications, such as Code Division Multiple Access (CDMA) or the Global System for Mobile Communications (GSM).

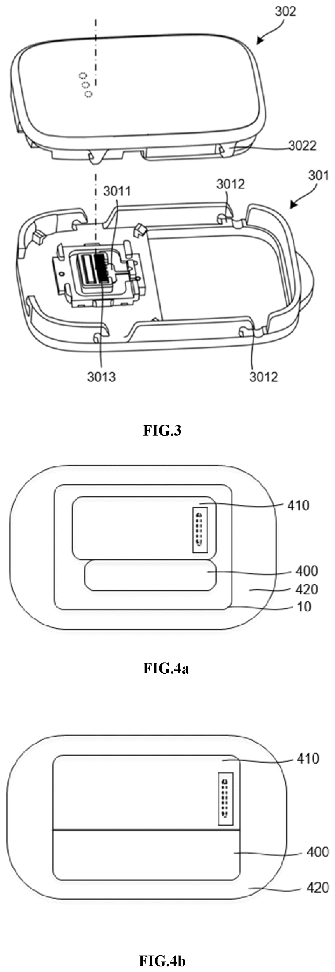

FIG.2 is a schematic diagram of the integrated continuous glucose monitoring device according to an embodiment of the invention. FIG.3 is a schematic diagram of the split continuous glucose monitoring device according to an embodiment of the invention.

The CGM comprising a sensor and a transmitter, mounted on the patient by means of an auxiliary mount, pierced subcutaneously. The sensor is used to collect analyte data in the human body and transmit the collected analyte content information. The transmitter is connected to the sensor, and it is used to receive the information on the blood glucose data, which is transmitted by the sensor that has been implanted under the skin, and convert the information into a wireless signal for outputting. Each CGM has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the CGM, or can also be set differently depending on the type of the CGM.

FIG.2 is a schematic diagram of the integrated continuous glucose monitoring device, i.e., the sensor and the transmitter have been integrated together prior to use, and is a single-use product, which is discarded after use, as shown in FIG. 2. The integrated continuous glucose monitoring device includes a sensor 201, a housing 202, and a transmitter (not shown in the figure) set inside the housing 202, the sensor 301 is used to monitor the patient's body fluid blood glucose data information, and transmit the said blood glucose data information to the transmitter through an internal circuit, which in turn sends it to a receiver. The identifier can be provided on the outer housing of the CGM or on the outer packaging or within the CGM.

FIG.3 is a schematic diagram of the split continuous glucose monitoring device, i.e., the sensor and the transmitter are two different parts prior to use, packaged separately, and only integrated together when in use, as shown in FIG. 3. The split continuous glucose monitoring device includes a base 301 and a transmitter 302, the base is provided with a sensor 3011, the transmitter 302 has a separate housing, the housings of the base 301 and the transmitter 302 are respectively provided with snap-in structures 3012 and 3022, the base 301 and the transmitter 302 are snapped into a whole through the snap-in structures when in use, the sensor 3011 is electrically connected to the transmitter 302 through the electrical connection 3013, the sensor 301 is used to monitor the patient's blood glucose data information, the blood glucose data information is transmitted to the transmitter 302 through the electrical connection 3013, and then sent to the receiver by the transmitter 302.

In one embodiment of the invention, both the sensor and the transmitter of the split continuous glucose monitoring device are single-use products, which are discarded after use, and therefore the identifier can be provided on the housing or the outer packaging of the sensor or the transmitter. In yet another embodiment of the invention, only the sensor of the split continuous glucose monitoring device is a single-use product and the transmitter is a reusable product, therefore, preferably, in this embodiment, the identifier is provided on the housing or the outer packaging of the transmitter, which reduces the frequency of tying the patient's information to the identifier and improves the patient experience. This will be described in more detail below.

When the identifier is set on the housing or outer packaging of the CGM or transmitter, it is set in a form that includes, but is not limited to, a QR code, a bar code, or an NFC tag.

FIG.4a is a schematic diagram of the integrated insulin pump according to an embodiment of the invention. FIG.4b is a schematic diagram of the split insulin pump according to an embodiment of the invention.

In this embodiment of the invention, the insulin pump is a patch-type insulin pump, i.e., an insulin pump that does not comprise a long catheter, includes an infusion mechanism and a control mechanism, and being integrally affixed to the surface of the user's skin by an adhesive patch, and the medication is infused directly from the reservoir to the subcutaneous area along the infusion needle.

Each insulin pump has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the insulin pump, or can also be set differently depending on the type of the insulin pump.

FIG.4a is a schematic diagram of the integrated insulin pump, i.e., the infusion mechanism 410 and the control mechanism 400 of the insulin pump are provided inside the same housing 10, and both are connected by wires and are affixed to a location on the user's skin by means of an adhesive patch 420, which is disposed of in its entirety after a single use. an identifier can be provided on the outer housing of the insulin pump or on the outer packaging or inside the insulin pump.

FIG.4b is a schematic diagram of the split insulin pump, i.e., the infusion mechanism 410 and the control mechanism 400 of the insulin pump are provided in two different housings, both of which are connected by waterproof plugs or directly snap together and electrically connected as a whole, an identifier can be provided on the outer housing of the infusion mechanism and/or the control mechanism or on the outer packaging or inside the insulin pump.

In one embodiment of the invention, both the infusion mechanism and the control mechanism of the split insulin pump are single-use products, which are discarded after use, and therefore the identifiers can be provided on the housing or on the outer packaging of the infusion mechanism and/or the control mechanism. In yet another embodiment of the invention, only the infusion mechanism of the split insulin pump is a single-use product, while the control mechanism is a reusable product, and therefore, preferably, in this embodiment, the identifier is set on the housing of the control mechanism or on the outer packaging, which can reduce the frequency of binding of patient information and identifiers, and improve the patient experience. This is described in more detail below.

When the identifier is set on the housing of the control mechanism or on the outer packaging, it is set in a form that includes, but is not limited to, a QR code, a bar code, or an NFC tag.

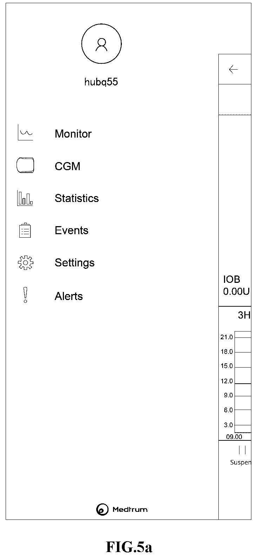

As mentioned earlier, considering the severity of illness in patients and the individual patient's physical health status, some patients can only need a CGM for continuous glucose monitoring, while others can need not only a CGM for continuous glucose monitoring, but also an insulin pump for drug infusion. When a doctor determines that a patient only needs to use a CGM for continuous glucose monitoring, and as the CGM involves only the monitoring of the patient's user's blood glucose, and the user's own use of the CGM does not pose a risk to the user's life and safety, the users can purchase the CGM by themselves. Before the CGM is installed on the surface of the user's skin, the user can search for and download the dedicated APP for controlling the CGM from the smartphone's app store, create a new account on the dedicated APP, and pair the patient's personal information with the information of the CGM to be worn, thus realizing the pairing and controlling of the smartphone and the CGM. The CGM and the insulin pump in the embodiment of the invention are developed and produced by the same manufacturer, and thus can be controlled by the same dedicated APP in the smartphone, and since the insulin pump is not required to be used by all patients, only CGM-related content is involved in the default main screen of the dedicated APP, as shown in FIG. 5a. On the one hand, it can simplify the interface of the APP and provide the user with a good visual experience, and on the other hand, prevent the user from mis-operation on the insulin pump, which can affect the normal use of the CGM.

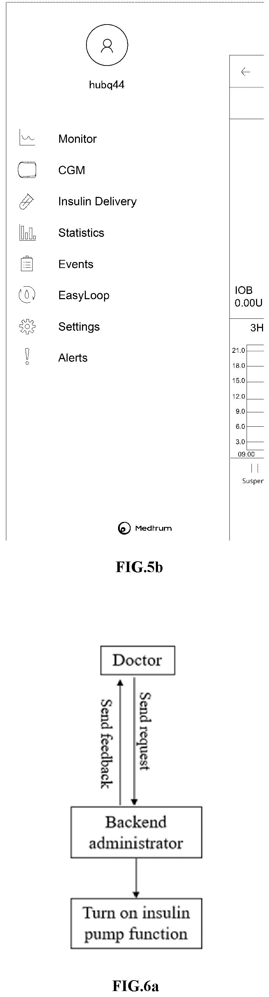

In one embodiment of the invention, when a doctor determines that a patient needs to use an insulin pump for drug infusion, as shown in FIG. 6a, the doctor sends a request to the backend administrator to add the patient's account to a whitelist to allow the patient to use the insulin pump function, and the backend administrator receives the request to add a whitelist sent by the doctor and adds the patient's account to a list of whitelists, and at the same time sends the doctor a feedback that the addition of the whitelist has been completed, and furthermore the backend administrator turns on directly the insulin pump function on the app interface that the patient is using. At this time, the APP interface changes from FIG. 5a to FIG. 5b, and the interface of FIG. 5b adds two function keys related to insulin infusion, "Insulin Delivery" and "Easyloop", compared with the interface of FIG. 5a. In another embodiment of the invention, the backend administrator does not directly turn on the insulin pump function of the APP interface used by the patient, but sends a security code to the patient's account, so that the patient can turn on the insulin pump function on the APP interface through the security code when needed or convenient.

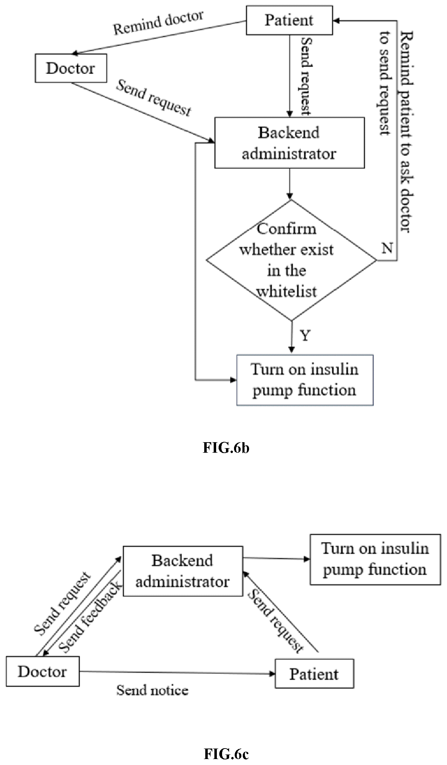

In another embodiment of the invention, when the doctor determines that the patient needs to use the insulin pump for drug infusion, as shown in FIG. 6b, the patient can send an request to turn on the insulin pump function directly to the backend administrator, and the backend administrator receives the request to turn on the insulin pump function sent by the patient and verifies that whether the patient's account is in the whitelist or not, and if the patient's account is in the whitelist, the backend administrator turns on the insulin pump function in the APP interface that the patient is using and the APP interface changes from FIG. 5a to FIG. 5b. If the patient's account is not in the whitelist, a feedback message is sent to the patient's account to remind the patient to ask the doctor to send an add whitelist request to the backend administrator. After the doctor sends the whitelist request to the background administrator, the background administrator can directly turn on the insulin pump function of the APP interface used by the patient. If no message is received from the background administrator within a certain period of time, such as 1min, 2min, 5min, then another request for turning on the insulin pump function to the backend administrator can be send again, or ask the doctor to send the request for adding whitelist to the backend administrator. In another embodiment of the invention, the backend administrator does not directly turn on the insulin pump function on the APP interface used by the patient, but instead sends a security code to the patient's account, which allows the patient to turn on the insulin pump function of the APP interface through the security code when needed or convenient.

In yet another embodiment of the invention, when the doctor determines that the patient needs to use the insulin pump for drug infusion, as shown in FIG. 6c, the doctor sends a request to the backend administrator to add the patient account to the whitelist to allow the patient to use the insulin pump function, and the backend administrator receives the request sent by the doctor to add a whitelist and adds the patient account to the whitelist, and at the same time sends the doctor a feedback that the whitelist addition has been completed, and further the doctor notifies the patient that the patient can apply to use the insulin pump function. After receiving the notification from the doctor, the patient sends an request for turning on the insulin pump function to the backend administrator, and after the backend administrator receives the request for turning on the insulin pump function sent by the patient, the backend administrator directly turns on the insulin pump function on the APP interface used by the patient. In another embodiment of the invention, after the backend administrator receives the request to turn on the insulin pump function sent by the patient, the backend administrator can also first verify whether the patient account exists in the whitelist or not, and if it is determined that the patient account is in the whitelist, the backend administrator turns on the insulin pump function on the APP interface used by the patient. In another embodiment of the invention, the backend administrator does not directly turn on the insulin pump function on the APP interface used by the patient, but instead sends a security code to the patient's account, through which the patient can turn on the insulin pump function on the APP interface when needed or convenient.

When the doctor determines that the patient no longer needs to turn on the insulin pump function, the patient can turn off the insulin pump function on the APP by himself/herself, and the APP automatically sends a message to the backend administrator, who deletes the patient's account from the whitelist. the doctor and/or the patient can also send an request to the backend administrator to request to turn off the insulin pump function, and the backend administrator will turn off the insulin pump function on the APP and at the same time delete that patient account from the whitelist. When this patient needs to turn on the insulin pump function again, the patient account needs to be re-added to the whitelist by referring to one of methods as shown in FIG. 6a- FIG.6c.

It should be noted that it is necessary to ensure that the insulin pump is properly mounted on the skin before turning on the insulin pump function of the APP, and to pair the patient's personal information with the information of the insulin pump, so as to realize the pairing and controlling of the smartphone and the insulin pump. The personal information of the patient includes name, age, gender, cell phone number, etc., and the information of the CGM and/or insulin pump worn includes the identifier information of the CGM and/or insulin pump. At the same time, the smartphone uploads the personal information of the patient and the identifier information of the CGM and/or the insulin pump to a remote server, which can store the information uploaded by the smartphone and verify whether the identifier information of the CGM and/or the insulin pump is valid, and if a certain identifier information already exists in the remote server, the remote server sends an alert to the smartphone to remind the user that the CGM or insulin pump has been used and needs to be replaced. When the CGM and/or the insulin pump is mounted on the skin of the patient and successfully activated, the CGM and/or the insulin pump starts to work, the transmitter of the CGM sends the monitored blood glucose information to the smartphone and further uploads it to the remote server, and the control mechanism of the insulin pump receives the insulin infusion information and controls the infusion mechanism to infuse the insulin, and at the same time sends the infusion status to the smartphone and further uploads it to the remote server.

It should be noted that the CGM and insulin pump in the embodiment of the invention are developed and produced by the same manufacturer, and thus can be controlled by the same dedicated APP in the smartphone. Assuming that CGM or insulin pumps produced by other manufacturers also can be controlled by a dedicated APP in the smartphone, and the inconvenience to the user due to the use of different APPs for controlling the CGM and the insulin pump respectively can be avoided, and the user experience can be improved.

When the CGM and/or insulin pump worn by the patient needs to be replaced for reasons such as reaching the end of its life cycle or failing, the unique identifier information of the new CGM and/or insulin pump also needs to be updated by pairing with the patient's personal information via the smartphone and further uploaded to a remote server. The patient's personal information is entered manually, and the identifier information of the CGM and/or insulin pump can also be entered manually or by scanning a QR code, a barcode, or NFC tags on the housing or on the outer packaging of the CGM and/or the insulin pump.

When the CGM is split and the transmitter is reusable, the identifier of the CGM is set on the housing or packaging of the transmitter, so that the patient only needs to replace the sensor when replacing the CGM without replacing the transmitter, and the identifier of the CGM remains unchanged, and thus there is no need for updating the pairing of the identifier of the CGM with the personal information of the patient via a smartphone, and no need for uploading it to a remote server, and thus the number of operational steps can be reduced to improve the patient experience.

When the insulin pump is split and the control mechanism is reusable, the identifier of the insulin pump is set on the housing or packaging of the control mechanism, and the patient only needs to change the infusion mechanism when replacing the insulin pump without changing the control mechanism, and the identifier of the insulin pump remains unchanged, and thus there is no need for updating the pairing update of the identifier of the insulin pump with the personal information of the patient via the smartphone or in uploading it to a remote server, and thus the operational steps can be reduced and the patient experience can be improved.

The smartphone and the CGM and/or insulin pump, and the remote server communicate with each other via wireless communication, which can be via, for example, but not limited to, radio frequency (RF) communication (e.g., radio frequency identification (RFID), Zigbee communication protocols, WiFi, infrared, wireless Universal Serial Bus (USB), Ultra Wide Band (UWB), Bluetooth^{®} communication protocols, and cellular communication, such as Code Division Multiple Access (CDMA) or Global System for Mobile Communications (GSM). communications, such as Code Division Multiple Access (CDMA) or Global System for Mobile Communications (GSM). Preferably, the smartphone and the remote server communicate with each other via WiFi and/or cellular communication, and the smartphone and the CGM and/or insulin pump communicate with each other via Bluetooth^{®} communication protocol.

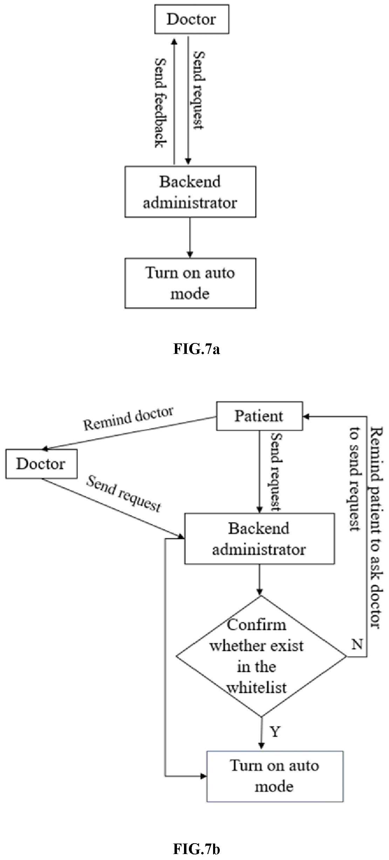

When the doctor determines that the patient can turn on the automatic mode, i.e. after the APP reads the current blood glucose value monitored by the CGM and the insulin information infused by the insulin pump, it can predict the future trend of blood glucose and control the infusion of the insulin pump based on the predicted trend of blood glucose, including increasing, decreasing, or stopping the infusion of insulin, in order to affect the blood glucose values, and form an automated closed-loop control cycle. As shown in FIG. 7a, the doctor sends a request to the backend administrator to add the patient account to the whitelist to allow the patient to use the auto mode function, and the backend administrator receives the add whitelist request sent by the doctor and adds the patient account to the whitelist, and at the same time sends the feedback to the doctor that the whitelist has been added, and furthermore directly turns on the auto mode function on the APP interface used by the patient.

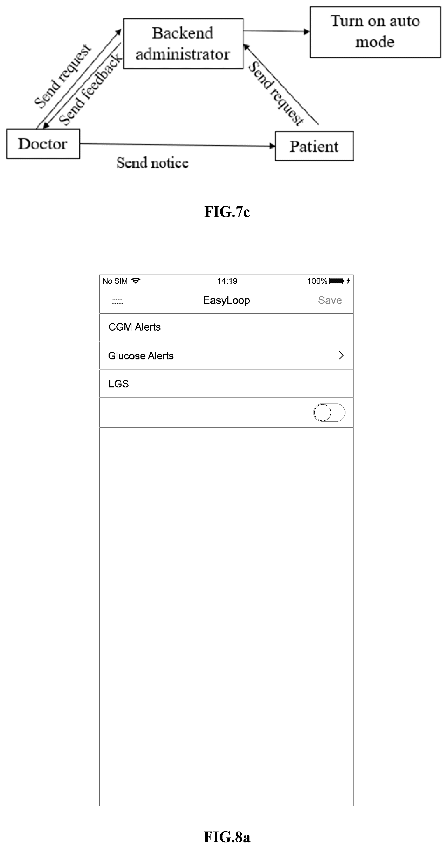

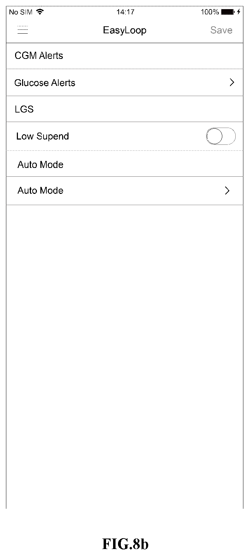

At this time, the APP interface changes from FIG. 8a to FIG. 8b, and the interface of FIG. 8b has added "Auto Mode", i.e., a function key related to the auto mode, compared with the interface of FIG. 8a. In another embodiment of the invention, the backend administrator does not directly turn on the Auto Mode function on the APP interface used by the patient, but sends a security code to the patient's account, so that the patient can turn on the Auto Mode function on the APP interface through the security code when needed or convenient.

In another embodiment of the invention, when the doctor determines that the patient needs to use the insulin pump for drug infusion, as shown in FIG. 7b, the patient can send a request to turn on the auto mode function directly to the backend administrator, and the backend administrator, upon receipt of the request sent by the patient to turn on the auto mode function, verifies whether the patient's account is in the whitelist or not, and if the patient's account is in the whitelist, the backend administrator turns on the auto mode function on the APP interface that is used by the patient, and the APP interface changes from FIG. 8a to FIG. 8b. If the patient's account is not in the whitelist, a feedback message is sent to the patient's account to remind the patient to ask the doctor to send an add whitelist request to the background administrator. After the doctor sends the whitelist request to the backend administrator, the backend administrator can directly turn on the auto mode function on the APP interface used by the patient. If no message is received from the backend administrator within a certain period of time, such as 1min, 2min, 5min, another request for turning on the insulin pump function to the backend administrator can be sent again, or ask the doctor to send the request for adding whitelist to the backend administrator. In another embodiment of the invention, the backend administrator does not directly turn on the auto mode function on the APP interface used by the patient, but instead sends a security code to the patient's account, through which the patient can turn on the auto mode function on the APP interface when needed or convenient.

In yet another embodiment of the invention, when the doctor determines that the patient needs to use the insulin pump for drug infusion, as shown in FIG. 7c, the doctor sends a request to the backend administrator to add the patient account to the whitelist to allow the patient to use the auto mode function, and the backend administrator receives the request to add a whitelist sent by the doctor and adds the patient account to the whitelist, and at the same time sends a feedback to the doctor that the whitelist has been added, and furthermore the doctor notifies the patient that he/she can request to use the auto mode function. After receiving the notification from the doctor, the patient sends an request for turning on the auto mode function to the backend administrator, and after the backend administrator receives the request for turning on the auto mode function sent by the patient, the backend administrator directly turns on the auto mode function on the APP interface used by the patient. In another embodiment of the invention, after the backend administrator receives the request for turning on the auto mode function sent by the patient, the backend administrator can also first verify whether the patient account exists in the whitelist or not, and if it is determined that the patient account is in the whitelist, the backend administrator turns on the auto mode function on the APP interface used by the patient. In another embodiment of the invention, the backend administrator does not directly turn on the auto mode function on the APP interface used by the patient, but instead sends a security code to the patient's account, through which the patient can turn on the auto mode function on the APP interface when needed or convenient.

When the doctor determines that the patient no longer needs to turn on the auto mode function, the patient can turn off the auto mode function on the APP by themselves, and the APP automatically sends a message to the backend administrator, who deletes the patient account from the whitelist. Or the doctor and/or the patient can also send a request to the backend administrator to turn off the auto mode function, and the backend administrator turns off the auto mode function on the APP, and at the same time deletes the patient account from the whitelist. When this patient needs to turn on the auto mode function again, the patient account needs to be re-added to the whitelist by referring to one of the methods as shown in FIG. 7a-FIG. 7c.

In embodiments of the invention, the security code sent by the backend administrator when applying for insulin pump function and auto mode function can be any one or combination of a series of numeric characters, alphabetic characters, and other symbols, as well as a series of taps, a series of inputs, complex or simple gestures (e.g., swipes or other movements on a touch screen, drawing images), and the like. In some cases, the security code can also include a quiz or set of questions. Each security code sent by the backend administrator is randomized.

Given the considerable variation in dietary habits across different regions and age groups, a standardized control system can lead to suboptimal glycemic management for certain individuals, when the patient's auto mode function is turned on, the patient is asked to enter a pass code to access the different auto mode interfaces.

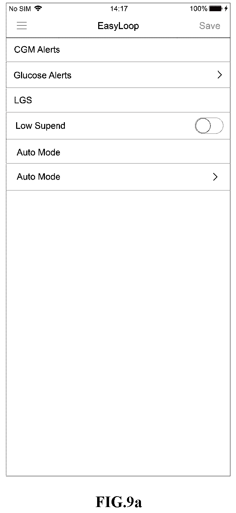

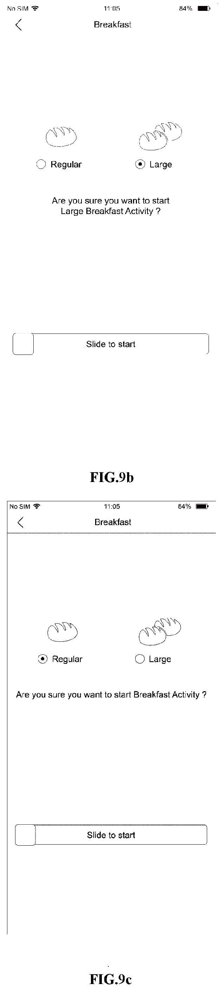

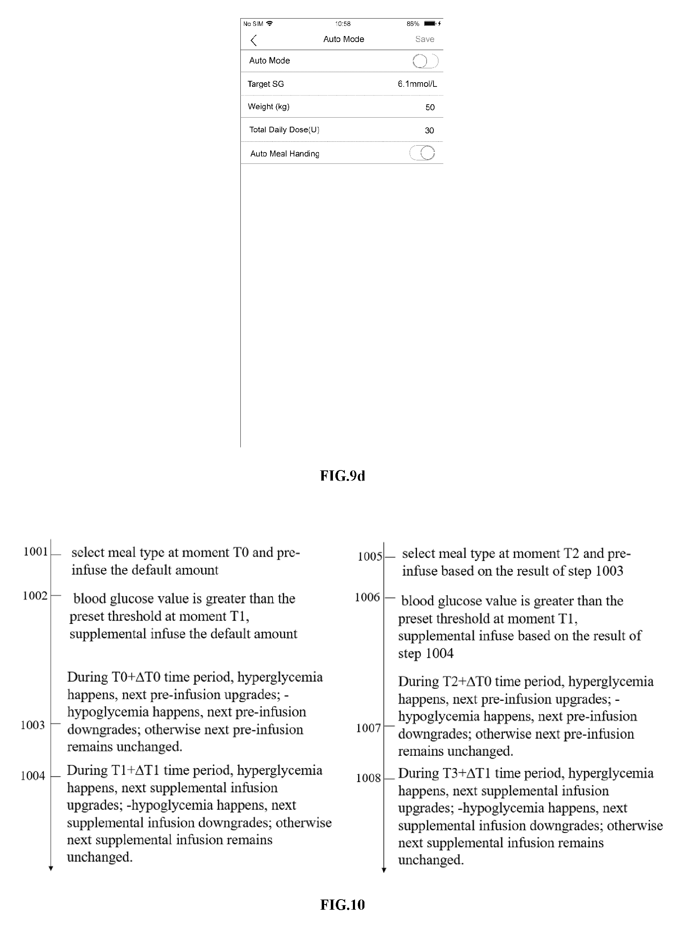

For people with high carbohydrate consumption, entering the pass code to access the high carbohydrate mode, and the interface of the auto mode is shown in FIG. 9a, a large meal option displays on the interface of the high carbohydrate mode, the patient can choose whether to turn on the function of the Large Meal option, after the patient turns on the function of the Large Meal option, two options of "Regular" and "Large" will be displayed on the infusion page, as shown in Fig. 9b and FIG. 9c. When the patient selects "regular ", the system will inject at least the amount of insulin corresponding to the amount of regular carbohydrates. when the patient selects "Large ", the system will inject at least the amount of insulin corresponding to a larger amount of carbohydrates. For people with normal carbohydrate consumption, after entering the pass code, the system enters the normal carbohydrate mode, the interface of the auto mode is shown in FIG. 9d, the interface of the normal carbohydrate mode does not have the option of "Large Meal", and the default is the " Regular " mode, and the system will infuse at least the amount of insulin corresponding to the amount of regular carbohydrates.

In embodiments of the invention, the pass code can be a small set of questions, such as "Are you a carb enthusiast?", "Is your age in the A-B range?", "What is your gender?", "Where do you live?", "What are your fitness preferences?", "Are there any special medical conditions?", "Have you uses the non-auto mode before turning on the auto mode?" etc. Based on the patient's answers, the system automatically determines whether the patient is a high carbohydrate consumer. In other embodiments of the invention, the pass code is communicated to the patient by a doctor after diagnosing, or the doctor sends information about whether the patient is a high carbohydrate consumer while sending a whitelist request for the auto mode to the backend administrator, and the backend administrator automatically assigns the patient the corresponding pass code, which can be anyone or combination of a series of numeric characters, alphabetic characters, and other symbols, as well as a series of taps, a series of inputs, complex or simple gestures (e.g., swipes or other movements on a touch screen, drawing images), and the like, and the backend administrator can send the pass code to the patient when turning on the auto mode, or send the pass code to the patient at the same time as sending the security code, or send the pass code to the patient after confirming that the patient has turned on the auto mode via the security code. In the embodiment of the invention, both the security code and the pass code are randomly generated, and their generation rules can or cannot be the same, and preferably, the generation rules of the security code and the access code are not the same, so as to avoid confusion of the patient and thus causing disturbance to the patient.

In embodiments of the invention, the system or the doctor's judgment of whether the patient is a high carbohydrate consumer can be the result of a comprehensive judgment based on a plurality of conditions, such as the patient's gender, age, dietary habits, exercise habits, health status, insulin sensitivity, carbohydrate sensitivity, and results of the use of the non-auto mode, and when the patient has a record of the use of the non-auto mode, the result of the non-auto mode is used as the primary basis for the judgment.

When the patient selects a meal, including "Regular" and "Large" meals, the auto mode uses the drug infusion strategy of pre-infusion and supplemental infusion, as shown in FIG 10. In pre-infusion and supplemental infusion strategy, the estimated meal size corresponding to the pre-infusion amount and supplemental infusion amount were categorized into different levels for both "Regular" and "Large" meals, as shown in following Table 1:

| Meal option | Estimated meal size for pre-infusion | Estimated meal size for supplemental infusion |
|---|---|---|
| Regular | Small (default) | Small (default) |
| | Middle | Middle |
| | Big | Big |
| Large | Small (default) | Small (default) |
| | Middle | Middle |
| | Big | Big |

Wherein, the insulin amount for the pre-infusion is at least related to the actual blood glucose value or its rate of change at the time of pre-infusion, and the estimated meal size for pre-infusion. In other embodiments of the invention, the insulin amount for the pre-infusion can also be related to the IOB in the body. Similarly, the insulin amount for the supplemental infusion is at least related to the actual blood glucose value or its rate of change at the time of supplemental infusion, and estimated meal size for the supplemental meal. In other embodiments, the insulin amount for supplemental infusion can also be related to the IOB in the body. In embodiments of the invention, the meal size is the amount of carbohydrate contained in the meal.

The estimated meal sizes (Small, Middle, Big) for the pre-infusion and the supplemental infusion are independent parameters. For the same level, the estimated meal size for the pre-infusion is greater than the estimated meal size for the supplemental infusion, but there is not necessarily a fixed correspondence between the two, and the system can be set according to the actual needs. For the Large meal, the minimum value of the estimated meal size for the pre-infusion is not less than the maximum value of the Regular meal, and the minimum value of the estimated meal size for the supplemental infusion is not less than the maximum value of the Regular meal. In embodiments of the invention, in pre-infusion, the range of estimated meal sizes for Regular meals that can be 40-80g, 30-60g, etc., and for Large meals that can be 80-120g, 60-100g, etc., and then according to certain rules to divide for Big, Middle, Small, or more levels, respectively. In supplemental infusion, the range of estimated meal sizes for Regular meals that can be 20-40g, 10-30g, etc., and for Large meals that can be 40-60g, 30-50g, etc., and then according to certain rules to divide for Big, Middle, Small, or more levels, respectively. In other embodiments of the invention, the range of estimated meal sizes for Regular and Large meals at the time of pre-infusion or at the time of supplemental infusion can also be any other reasonable range, which is then according to certain rules to divide for Big, Middle, Small, or more levels, respectively.

FIG. 10 is a schematic diagram of the process of an infusion strategy for pre-infusion and supplemental infusion according to embodiments of the invention.

Step 1001, the patient selects a meal type at the moment of T0, and a default insulin amount is pre-infused, and the pre-infused default insulin amount can be corresponding to any one of the estimated meal sizes for pre-infusion, Big, Middle, or Small, and preferably, the pre-infused default insulin amount is corresponding to the Small size, and selecting a small amount of the initial pre-infused insulin amount can prevent too much insulin from being infused, and reduces the risk of causing hypoglycemia.

Step 1002, at the moment T1, comparing the current blood glucose value monitored by the CGM with a preset blood glucose threshold, e.g., 140, 150, 160, 170, 180, 190, 200 mg/mL, etc., if the current blood glucose value is greater than the preset blood glucose threshold, the default supplemental insulin amount is infused, otherwise, the supplemental infusion amount is not infused, and the default supplemental insulin amount can be corresponding to any one of the estimated meal sizes for supplemental infusion, Small, Middle, or Big, and preferably, the default supplemental insulin amount is corresponding to the small size, and selecting a small amount of the initial supplemental infused insulin amount can prevent too much insulin from being infused, and reduces the risk of causing hypoglycemia. The T1 moment is a moment later than the T0 moment, such as 1h, 1.5h, 2h, 2.5h, 3h, etc. In other embodiments of the invention, the rate of change of blood glucose at the T1 moment can also be combined to determine whether to administer a supplemental infusion.

Step 1003, within ΔT0 time period after the T0 moment, e.g., within 3h, 4h, 5h, etc., if hyperglycemia happened, the next pre-infusion will be upgraded, i.e., the amount of insulin corresponding to a bigger estimated meal size will be infused, at the same time, taking into account the actual glucose value or rate of change of glucose, or IOB at the moment of the next pre-infusion. If hypoglycemic happened, the next pre-infusion will be downgraded, i.e., the amount of insulin corresponding to a smaller estimated meal size will be infused, at the same time, taking into account the actual glucose value or rate of change of glucose, or IOB at the moment of the next pre-infusion. If no hyperglycemia or hypoglycemic happened, the level of the next pre-infusion will be maintained, i.e., the amount of insulin corresponding to the same estimated meal size will be is infused, at the same time, taking into account the actual glucose value or rate of change of glucose, or IOB at the moment of the next pre-infusion.

It should be noted that in embodiments of the invention, an adjustment in the infusion level implies an adjustment in the level of the estimated meal size at the time of the infusion, at the same time, taking into account the actual blood glucose value or rate of change of blood glucose, or IOB at the moment of the infusion, and therefore, in embodiments of the invention, an adjustment in the level of the estimated meal size implies an adjustment in the level of the infusion, i.e., the level of the infusion and the level of the estimated meal size can be understood to be the same.

Step 1004, within ΔT1 time period after the T1 moment, e.g., within 3h, 4h, 5h, etc., if hyperglycemia happened, the next supplemental infusion will be upgraded, i.e., the amount of insulin corresponding to a bigger estimated meal size will be infused, at the same time, taking into account the actual glucose value or rate of change of glucose, or IOB at the moment of the next s supplemental infusion. If hypoglycemic happened, the next supplemental infusion will be downgraded, i.e., the amount of insulin corresponding to a smaller estimated meal size will be infused, at the same time, taking into account the actual glucose value or rate of change of glucose, or IOB at the moment of the next supplemental infusion. If no hyperglycemia or hypoglycemic happened, the level of the next supplemental infusion will be maintained, i.e., the amount of insulin corresponding to the same estimated meal size will be infused, at the same time, taking into account the actual glucose value or rate of change of glucose, or IOB at the moment of the next supplemental infusion.

Step 1005, the patient selects the meal type at the T2 moment, and pre-infuses the amount of insulin corresponding to a bigger, smaller, or unchanged estimated meal size based on the results of step 1003, and taking into account the actual blood glucose value or the rate of change of blood glucose or the IOB at the moment of T2.

Step 1006, at the moment T3, comparing the current blood glucose value monitored by the CGM with a preset blood glucose threshold, e.g., 140, 150, 160, 170, 180, 190, 200 mg/mL, etc., if the current blood glucose value is greater than the preset blood glucose threshold, supplemental infuses the amount of insulin corresponding to a bigger, smaller, or unchanged estimated meal size based on the results of step 1004, and taking into account the actual blood glucose value or the rate of change of blood glucose or the IOB at the moment of T3. if the current blood glucose value is not greater than the preset blood glucose threshold, no supplemental infuse is performed. In other embodiments of the invention, the rate of change of blood glucose at the T3 moment can also be combined to determine whether to administer a supplemental infusion.

Step 1007, within ΔT2 time period after the T2 moment, e.g., within 3h, 4h, 5h, etc., if hyperglycemia happened, the next pre-infusion will be upgraded, i.e., the amount of insulin corresponding to a bigger estimated meal size will be infused, at the same time, taking into account the actual glucose value or rate of change of glucose, or IOB at the moment of the next pre-infusion. If hypoglycemic happened, the next pre-infusion will be downgraded, i.e., the amount of insulin corresponding to a smaller estimated meal size will be infused, at the same time, taking into account the actual glucose value or rate of change of glucose, or IOB at the moment of the next pre-infusion. If no hyperglycemia or hypoglycemic happened, the level of the next pre-infusion will be maintained, i.e., the amount of insulin corresponding to the same estimated meal size will be infused, at the same time, taking into account the actual glucose value or rate of change of glucose, or IOB at the moment of the next pre-infusion.

Step 1008, within ΔT1 time period after the T3 moment, e.g., within 3h, 4h, 5h, etc., if hyperglycemia happened, the next supplemental infusion will be upgraded, i.e., the amount of insulin corresponding to a bigger estimated meal size will be infused, at the same time, taking into account the actual glucose value or rate of change of glucose, or IOB at the moment of the next s supplemental infusion. If hypoglycemic happened, the next supplemental infusion will be downgraded, i.e., the amount of insulin corresponding to a smaller estimated meal size will be infused, at the same time, taking into account the actual glucose value or rate of change of glucose, or IOB at the moment of the next supplemental infusion. If no hyperglycemia or hypoglycemic happened, the level of the next supplemental infusion will be maintained, i.e., the amount of insulin corresponding to the same estimated meal size will be infused, at the same time, taking into account the actual glucose value or rate of change of glucose, or IOB at the moment of the next supplemental infusion.

Repeat steps 1005-1008 when pre-infusion and supplemental infusion is required at the next moment.

Generally, according to the patient's dietary habits, and in order to maintain the stability of the patient's blood glucose level, the patient chooses the same meal type at the moment of T2 and T0, i.e., if the patient chooses Regular at the moment of T0, he/she also chooses Regular at the moment of T2. If Large is selected at the moment of T0, Large meal is also selected at T2, so that the next pre-infusion insulin selection can be dependent on the results of the previous pre-infusion insulin selection, and the next supplemental insulin infusion selection can also be dependent on the results of the previous supplemental insulin infusion selection, except that, if the patient's choice of the next meal is inconsistent with that of the previous one, e.g., when the next meal selection is not the same as the previous one for the first time, the patient is reverted back to the initial default level for the next pre-infusion and supplemental infusion in order to prevent inaccuracy of the amount of insulin infusion due to change in the mode of the meal. When the meal choice is different again, i.e., when the next two choices revert back to being the same as the previous choice, the levels at the next two pre-infusions and supplemental infusion are the same as the pre-infusion and supplemental infusion levels of the previous one, based on the same infusion levels as the previous one, which results in a more accurate insulin infusion at the current meal and better control of the patient's blood glucose level. For example, assuming that the patient's previous five choices were all Regular, wherein the pre-infusion and supplemental infusion levels were both Middle, and that when the choice for the 6th choice was Large (the choices are different for the first time), the pre-infusion and supplemental infusion levels at the 6th choice were the default levels. and that when the patient's choice for the 7th choice reverted back to Regular (the choices are different again), the pre-infusion and supplemental infusion levels at the 7th choice were the same as those that had been used at the time of the pre-infusion and supplemental infusion at the 5th choice, i.e., that is Regular, and the level is Middle.

Here, it should be noted that if the current pre-infusion amount corresponding to the estimated meal size is already the biggest estimated meal size in the selected meal type, and if hyperglycemia still occurs during the ΔT0 time period after the T0 or T2 moments, the next pre-infusion amount will not be upgraded, and the amount of insulin corresponding to the biggest (Big in here) estimated meal size will still be pre-infused, taking into account the actual glycemic value or the rate of change in glycemia or the IOB at the moment of the next pre-infusion. Similarly, if the current pre-infusion amount corresponding to the estimated meal size is already the smallest estimated meal size in the selected meal type, and if hypoglycemia still occurs during the ΔT0 time period after the T0 or T2 moments, the next pre-infusion amount will not be downgraded, and the amount of insulin corresponding to the smallest (Small in here) estimated meal size will still be pre-infused, taking into account the actual glycemic value or the rate of change in glycemia or the IOB at the moment of the next pre-infusion. That is, it limits the safe infusion boundaries for insulin infusion and effectively prevents hyperglycemia and hypoglycemia.

If the current supplemental infusion amount corresponding to the estimated meal size is already the biggest estimated meal size in the selected meal type, and if hyperglycemia still occurs during the ΔT1 time period after the T1 or T3 moments, the next supplemental infusion amount will not be upgraded, and the amount of insulin corresponding to the biggest (Big in here) estimated meal size will still be supplemental infused, taking into account the actual glycemic value or the rate of change in glycemia or the IOB at the moment of the next supplemental infusion. Similarly, if the current supplemental infusion amount corresponding to the estimated meal size is already the smallest estimated meal size in the selected meal type, and if hypoglycemia still occurs during the ΔT1 time period after the T1 or T3 moments, the next supplemental infusion amount will not be downgraded, and the amount of insulin corresponding to the smallest (Small in here) estimated meal size will still be supplemental infused, taking into account the actual glycemic value or the rate of change in glycemia or the IOB at the moment of the next supplemental infusion. That is, it limits the safe infusion boundaries for insulin infusion and effectively prevents hyperglycemia and hypoglycemia.

In other embodiments of the invention, the corresponding estimated meal size at the time of pre-infusion and at the time of supplemental infusion are not necessarily both leveld, i.e., the estimated meal size at the time of pre-infusion can be only the default level, while at the time of supplemental infusion, the estimated meal size is of three different levels of Big, Middle and Small, respectively, and therefore at the pre-infusion stage, only the amount of insulin corresponding to the default meal size is infused. or the estimated meal size at the time of pre-infusion has three different levels of Big, Middle and Small, while at the time of the supplemental infusion, the estimated meal size is only the default level, and thus at each time of supplemental infusion only the amount of insulin corresponding to the default meal size is infused.

Similarly, the level settings for the estimated meal size corresponding to pre-infusion and supplemental infusion are not necessarily the same in Large and Regular, i.e., for each meal type, the estimated meal size corresponding to pre-infusion and supplemental infusion can be selected to be leveld or ungraded (the default size), which can be set according to the patient's actual needs.

In other embodiments of the invention, the high carbohydrate mode and the normal carbohydrate mode also include a small carbohydrate mode, such as the small carbohydrate mode can be selected when the patient eats a snack, and when the patient selects the small carbohydrate mode, the system performs the insulin infusion based on the default estimated meal size because of the small range of the carbohydrate content in the small carbohydrate, and at the same time, taking into account the actual glycemic value or rate of change of the blood glucose, or IOB of the patient when he/she eats the snack, and the default estimated meal size in the small carbohydrate mode is less than the lowest level of the estimated meal size in the normal mode.

In an embodiment of the invention, a small meal mode is further provided in the system, i.e. snack mode, when the patient selects the snack mode, since the range of carbohydrate content in snacks is relatively small, the system therefore performs insulin infusions on a default estimated meal size basis, taking into account the patient's actual blood glucose value or rate of change of blood glucose and IOB in the body when consuming the snack, and the default estimated meal size in the snack mode is less than the lowest level estimated meal size in the conventional mode.

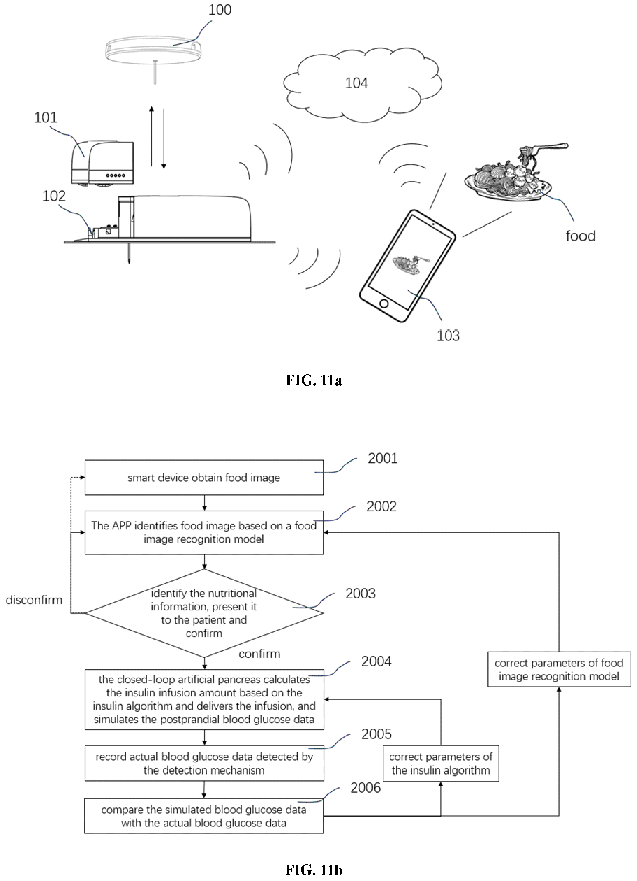

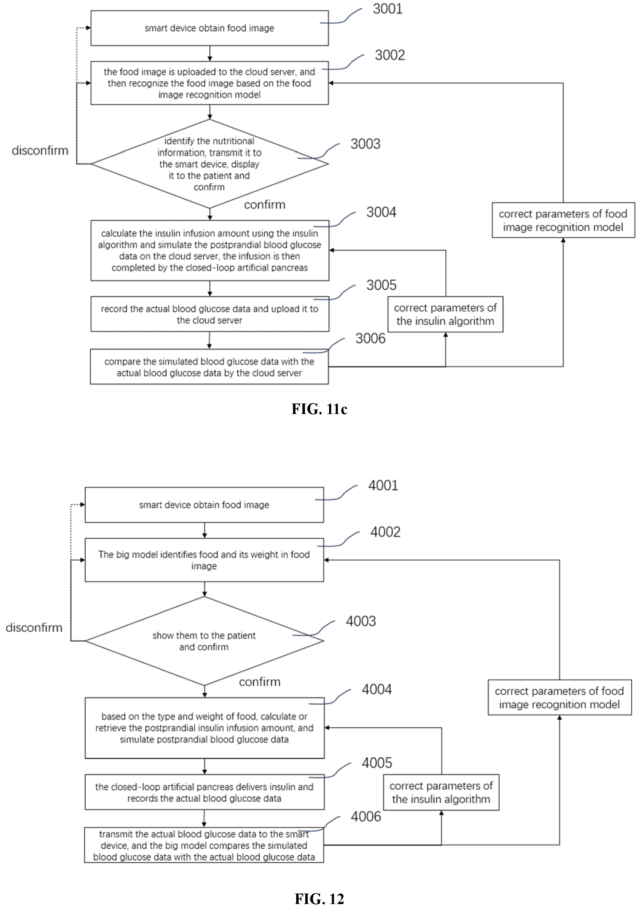

FIG. 11a is a schematic diagram of a closed-loop infusion system based on food image recognition according to an embodiment of the invention. FIG. 11b is a schematic diagram of a closed-loop infusion method based on food image recognition according to an embodiment of the invention. FIG. 11c is a schematic diagram of a closed-loop infusion method based on image recognition of food according to another embodiment of the invention.

In other embodiments of the invention, the amount of insulin infused during a meal can be further optimized to obtain a more accurate amount of insulin infused by quantifying the food to determine the amount of nutrients such as carbohydrate, fat and protein.

In an embodiment of the invention, incorporating an imaging mechanism 103 in the system, the imaging mechanism 103 can be used to acquire food images, the food images are input to the program mechanism 101 or the cloud server 104, and the food images are quantitatively analyzed to determine type and weight data of foods, such as potatoes 500g, noodles 340g, beef 220g, milk 450g, nutrient compositions of the foods are determined according to the type of foods, and nutrient contents of the respective foods are determined according to their weights. The cloud server 104 can be a large server disposed in a public network for storing and computing data.

Specifically, in some embodiments of the invention, the imaging mechanism 103 is an image acquisition device independent of the closed-loop artificial pancreas, with the development and popularity of closed-loop artificial pancreas technology, a smart device can be accessed into a closed-loop artificial pancreas, assist in completing blood glucose testing and insulin infusion for a patient, smart devices such as cell phones, tablets, head-worn augmented reality devices, and the like, the camera function is generally available, the patient can use the camera function of the smart device to take images of food before eating, acquire food images, and since the smart device can be iterated regularly, its image acquisition software and hardware satisfy the daily food image acquisition, the performance is superior, the captured food image is clear, and the quantitative analysis of food images is helpful.

In other embodiments of the invention, the imaging mechanism 103 can be a sub-module of the closed-loop artificial pancreas. For example, a camera function can be added to the PDM to acquire food images, thus eliminating the need for additional image acquisition equipment and saving costs for the patient.

Whichever device is used to take images of food is referred to in embodiments of the invention as imaging mechanism 103.

In an embodiment of the invention, the imaging mechanism 103 is wired or wirelessly connected into the closed-loop artificial pancreas system and data interacts with at least one of the detection mechanism 100, the program mechanism 101 and the infusion mechanism 102 to transmit food image data into the closed-loop artificial pancreas. Alternatively, after acquiring food images, the imaging mechanism 103 directly performs data analysis on the food images, determines type and weight of foods, then transmits the type and weight of foods into the closed-loop artificial pancreas to determine nutrient content, the system determines insulin infusion amount in combination with blood glucose data and infuses. Alternatively, the imaging mechanism 103 after acquiring food images, performs data analysis on the food images, determines type and weight of foods, then determines nutrient content according to type and weight of foods, then transmits nutrient data into the closed-loop artificial pancreas, the system determines insulin infusion amount in combination with blood glucose data and infuses. Alternatively, the imaging mechanism 103 after acquiring the food image, performing data analysis on the food image, determining the type and weight of the food, then determining the nutrient content according to the type and weight of the food, determining the insulin infusion amount in combination with the blood glucose data, then transmitting the insulin infusion amount data to the closed-loop artificial pancreas, and the system performing infusion according to the insulin infusion amount data.

In an embodiment of the invention, after the imaging mechanism 103 acquires the food image, the food image can be subjected to quantitative analysis directly in the imaging mechanism 103, the food image can also be transmitted into the closed-loop artificial pancreas, the food image can be subjected to quantitative analysis by the system, the food image can also be transmitted into the cloud server 104, the food image can be subjected to quantitative analysis by the cloud server 104.

In an embodiment of the invention, the food image can be quantitatively analyzed by the deep learning food image recognition model, for example, identifying features such as color (R\G\B channel), shape, hierarchy, texture, projection, etc. in the food image, in combination with a photographing distance feature of the imaging mechanism 103 with respect to the food, and determining the type and weight of the food. In order to more accurately identify the food image, it is also possible to access a food image library, extract each food in the food image into the food image library, align with the images in the food image library, and determine the kind of the food. After the type and weight of the food are determined, the type and weight of the food are transmitted to a food nutrition library to determine the nutrients and contents of the food.

In an embodiment of the invention, the food nutrition library can record only the various nutritional contents of the various foods, as shown in Table 2.1, and the various nutrient contents are calculated by reading the food nutritional contents of the food nutrition library after identifying the type and weight of the food in the food image. The food nutrient library can also record the nutrient content of each food contained in a given weight of food, as shown in Table 2.2, and the nutrient content of each food in the food image can be obtained by directly reading the food nutrient library after identifying the type and weight of the food in the food image.

**Table 2.1 Food Nutrition Library**

| Food section | Nutrient content /% | | |
|---|---|---|---|
| | Carbohydrate | Fat | Protein |
| potato | α | β | γ |
| beef | α' | β' | γ' |
| noodle | α" | β" | γ" |
| milk | α‴ | β‴ | γ‴ |
| ... | | | |

In an embodiment of the invention, 500g of potatoes are identified in the food image and the potato has a carbon content of α, a fat content of β, and a protein content of γ recorded in the food nutrient library, then the potatoes provide a carbon content of 500g*α, a fat content of 500g*β, and a protein content of 500g*γ in the food image. If beef 250g is also identified in the same food image and beef has a carbon content of α', a fat content of β' and a protein content of γ' recorded in the food nutrient library, then beef provides a carbon content of 250g*α', a fat content of 250g*β' and a protein content of 250g*γ' in the food image. Then it was finally identified that the total carbohydrate in the food image was 500 g*α+250 g*α', the total fat was 500 g*β+250 g*β', and the total protein was 500 g*γ+250 g*γ'. Predicting a patient simulated blood glucose profile from these identified aggregate nutrient contents, and calculating a postprandial insulin infusion amount, and completing a corresponding insulin infusion. The above data is merely exemplary. In calculating the amount of insulin infused, algorithms such as PID, MPC, neural networks can be used to predict the patient's simulated blood glucose profile.

**Table 2.2 Food Nutrition Library**

| Food section | weight/g | Nutrient content /mg | | |
|---|---|---|---|---|
| | | Carbohydrate | Fat | Protein |
| potato | 100 | - | - | - |
| | 200 | - | - | - |
| | 300 | - | - | - |
| | 400 | a | b | c |
| | ... | | | |
| beef | 100 | - | - | - |
| | 200 | d | e | f |
| | 300 | - | - | - |
| | 400 | - | - | - |
| | ... | | | |
| ... | | | | |

In the present embodiment, 400 g of potatoes and 200 g of beef are identified in the food image, the potato carbon content is recorded in the food nutrient library as a (mg), the fat content is b (mg), the protein content is c (mg), the beef carbon content is recorded as d (mg), the fat content is e (mg), and the protein content is f (mg), then the total carbon content in the food image is finally identified as a+d (mg), the fat content is b+e (mg), and the protein content is c+f (mg). Predicting a patient simulated blood glucose profile based on these identified aggregate nutrient contents, and calculating a postprandial insulin infusion amount, and completing a corresponding insulin infusion. The above data is merely exemplary. In calculating the insulin infusion amount, insulin algorithms such as PID, MPC, neural networks predict the patient's simulated blood glucose profile.

In an embodiment of the invention, the food nutrition library can be stored in the smart device, the closed-loop artificial pancreas, or the cloud server 104, without limitation. Due to the limited storage space of the readable storage device, the food nutrition library will not record all specified weights, all type of food nutrition content, the system can display the closest food bar and its corresponding nutritional content for patient confirmation, when patients or their medical monitoring personnel are able to provide a more accurate nutritional content of food, it can be entered into the system through an interactive interface of the smart device or PDM and stored to update the recorded original data in the food nutrient library or add unrecorded original data to the food nutrient library, the unrecorded original data including food type, weight and nutrient content.

In embodiments of the invention, the food nutrient library can record not only the nutrient content of carbon, fat and protein, but also the nutrient content of cholesterol, vitamins, trace elements and the like, such as vitamin B, vitamin B, vitamin C, vitamin D, calcium, magnesium, iron, zinc, sodium, potassium and the like.

In an embodiment of the invention, after identifying the nutritional content in the food image, calculating a simulated blood glucose profile for each nutrient based on the total carbon water, the total fat, and the total protein, respectively, that is, carbohydrate glucose profile *l_{α},* fat glucose profile *l_{β},* and protein glucose profile *l_{γ},* which reflect the effects of postprandial carbohydrate, fat, and protein, respectively, on a patient's blood glucose. In calculating the simulated blood glucose profile, reference historical data and IOB can be combined. After obtaining the carbohydrate blood glucose profile Iα, the fat blood glucose profile *l_{β},* and the protein blood glucose profile *l_{γ},* the three curves were fitted to obtain the simulated blood glucose profile *1₁*. The simulated blood glucose profile *l₁* can be obtained by means of a linear fit of the carbohydrate blood glucose profile *l_{α},* the fat blood glucose profile *l_{β}* and the protein blood glucose profile *l_{γ}*: ${l}_{1} = g ∗ {l}_{α} + h ∗ {l}_{β} + i ∗ {l}_{γ}$

Where g, h, i are fit coefficients for each nutrient's blood glucose profile.

The simulated blood glucose profile *l₁* reflects the theoretical blood glucose change after identifying the nutrient content in the food image by the food image recognition model, calculating the insulin infusion amount and infusing to the patient.

In an embodiment of the invention, the fitting of the simulated blood glucose profile *l₁* lasts for a period of time, such as 2-5 hours, during which the detection mechanism 100 acquires the actual blood glucose profile *l₂* of the patient in real time. Comparing simulated blood glucose profile *l₁* data with actual blood glucose profile *l₂* data, optimization can be performed on the simulated blood glucose profile *l₁*, to improve the accuracy of the carbohydrate blood glucose profile *l_{α},* the fat blood glucose profile *l_{β}* and the protein blood glucose profile *l_{γ},* which can make the food image recognition model further learn, adjust the recognition algorithm parameter of the food image and the insulin algorithm parameter to improve the recognition accuracy rate of the food image and the calculation accuracy rate of the insulin infusion amount. In particular, after the end of the duration of fitting of the simulated blood glucose profile *l₁*, by comparing the difference of the simulated blood glucose profile *l₁* and the actual blood glucose profile *l₂* for this period of time, adjusting, optimizing the parameters of the carbohydrate blood glucose profile *l_{α},* the fat blood glucose profile *l_{β}* and the protein blood glucose profile *l_{γ},* this means that the contents of total carbon, total fat, and total protein identified by the pre-meal food image recognition model are adjusted and optimized, thereby modifying the insulin algorithm parameters to improve the accuracy and applicability of the insulin algorithm parameters. Also, according to the aforementioned comparison difference, the food image recognition algorithm parameters in the food image recognition model can be corrected so that the next food image recognition result will be corrected more accurately. The revision program for the food image recognition model algorithm parameters and the insulin algorithm parameters is repeatable, such revision program can be repeatedly performed to constantly iterate the food image recognition model algorithm and the insulin algorithm, such that the constantly iterative process is the deep learning process of the food image recognition model and the insulin algorithm.

In an embodiment of the invention, iterative revisions for the food image recognition model algorithm and the insulin algorithm is done based on the actual blood glucose profile *l₂* of the patient, upon infusion of insulin, living habit cannot be the same due to physiological characteristics of different patients, in an initial state, even if the food image recognition model recognizes the exact same nutrient content, and completing the corresponding insulin infusion, the actual blood glucose profile *l₂* eventually detected by the detection mechanism 100 will not be exactly the same, this causes the correction of the parameters of the food image recognition model and the insulin algorithm parameters to be different, and with repeated iterations of the correction procedure, the parameters of the food image recognition model will be more accurate, and the insulin algorithm parameters will gradually become unique parameters of the individual patient and no longer be applicable to other patients, and the insulin algorithm after repeated iterations of the parameters is also changed from the generalized insulin algorithm at the factory to the narrow insulin algorithm of the individual patient.

In an embodiment of the invention, the food image recognition model after repeated iterations of parameters, the insulin algorithm can be selected by the patient to be uploaded to the cloud server 104, either the patient agrees that the system automatically uploads the iterated food image recognition model and the insulin algorithm to the cloud server 104, or the food image recognition model and the insulin algorithm is run on the cloud server 104, and the patient can choose to agree to have the iterated food image recognition model and the insulin algorithm exposed back to the server. After sufficient insulin narrow algorithms are acquired, clusters of insulin narrow algorithms can be assembled. In a cluster, can be classified into different groups according to the identifiable features of the patient's physiological characteristics, living habits, etc. from which the model is derived, having common identifiable features within a group, for example, according to the patient's age or age interval, age intervals such as 0-10 years old, 10-15 years old, 15-18 years old, 18-20 years are divided into groups, or by patient sex, male and female patients were divided into two groups, wherein female patients can also be divided into two groups according to whether they are in pregnancy or not, or they can be grouped according to the patient's habitual daily exercise interval, 0-10 minutes, 10-30 minutes, 30-60 minutes, etc., or they can be grouped according to a plurality of common identifiable feature combinations, e.g., "10-15 years old patient with daily exercise interval 10-30 minutes", "15-18 years old male patient", "20-22 years old pregnant patient", etc. Identifiable features can also include personal insulin resistance, weight, genetic history, disease history, country, job nature, and the like. Since the patient from which the model was derived within each group has one or more common identifiable features, either algorithm within the group may, to some extent, be applicable to other patients with that identifiable feature, this provides convenience for patients with this identifiable feature, these patients, before meals, optionally whether to use food image recognition models and narrow insulin algorithms of other patients with the same identifiable features, this has higher accuracy and applicability than directly using the generalized insulin algorithm, and does not require multiple refinements of insulin algorithm parameters and image recognition models, higher recognition accuracy for food images, and improves the therapeutic effect of the closed-loop artificial pancreas system.

In an embodiment of the invention, a certain group of patients after using the food image recognition model of the group and the insulin algorithm of narrow sense, after one or several meals, the food image recognition model and narrow insulin algorithm is iterated, and the iterated food image recognition model and narrow insulin algorithm parameters can also be fed back into the patient's identifiable feature set. For example, one can identify a characteristic "male patient 16 years old", after he identifies the food image using the food image recognition model before eating, the insulin infusion is again done using the narrow insulin algorithm, 5 hours after a meal, he used the food image recognition model and the narrow insulin algorithm to complete the iterations, the patient chooses to publish the iterated food image recognition model and insulin narrow algorithm parameters to the background, then these parameters can be fed back to the group of "16 years old patients", also to the group of "male patients", and also to the group of "16 years old male patients".

In an embodiment of the invention, identifiable feature groups exist in parallel form independent of each other, for example, "male patient 16 years old" and "patient 16 years old" are two groups independent of each other in which applicable food image recognition model and narrow insulin algorithm parameters are stored. In these independent groups, the more identifiable features, the more accurate the food image recognition model and the narrow insulin algorithm in the group, the more suitable their parameters are for the patient. For example, one identifiable feature is "male patient 16 years old", he can find the food image recognition model and narrow insulin algorithm applicable to himself in either the "16 years old patients" or "16 years old male patients" group, obviously, the food image recognition model and narrow insulin algorithm he found in the "16 years old male patient" are more applicable to himself, since in the "16 yeas old patient" group, the food image recognition model and the narrow insulin algorithm also underwent learning of "female patients", patient-derived data that did not conform to the physiological state of the patient, this would obviously affect the insulin infusion amount calculation for "male patients".

In other embodiments of the invention, the identifiable feature sets are stored in the system or cloud server 104 in subsets, e.g., "16 yeas old male patients" is a lower subset of "16 yeas old patients", and in retrieving the "16 yeas old male patients" group, the "16 yeas old patients" group is retrieved first, and then the "male patients" group is retrieved in the "16 yeas old patients" group. Obviously, among the identifiable features, age and gender are two independent features, both of which can be used as lower subsets, and in searching the "16 yeas old male patients" group, it is also possible to search the "male patients" group first, and then search the "16 yeas old patients" group in the "male patients" group. Both of the above approaches can ultimately point to the group of "16 years old male patients", i.e. "16 years old patients" and "male patients" can be subsets of each other.

In an embodiment of the invention, the subset of identifiable features is oriented to an individual patient or patient population, the catalogue of which is editable. It is not possible for the manufacturer to pre-store all identifiable features in the system or cloud server 104 for the patient to select in an exhaustive manner, thus, the patient cannot be able to retrieve their applicable identifiable features, wherein case the patient can edit, add, modify, or delete the subset of identifiable features, or apply to the server to add, modify, or delete the subset of identifiable features.

In an embodiment of the invention, due to the objective certainty of the nutrient content in the food images, the food image recognition model parameters after multiple iterations will tend to be consistent, and the food image recognition model parameters provided by each patient can be used to refine the food image recognition model and the food nutrient library. Refining the food image recognition model through deep learning by means of big data, recognition accuracy of food images can be improved, which is beneficial for development of a closed-loop artificial pancreas system.

Referring to Figs. 11a and 11b, in an embodiment of the invention, at step 2001, a patient while using a closed-loop artificial pancreas system with food image recognition, a food image needs to be taken first for the food currently ready to eat by means of the imaging mechanism 103 of the smart device, considering that the food can be dispersed in a plurality of utensils as the patient eats, the patient can take one or more images of the food in each utensil, and can take different angles when taking the plurality of images of the food, so that portions of the food that can be occluded can be identified. The food image recognition model can recognize food repeatedly photographed by the patient by judging elements such as color, shade, shape, and size of food in the food image, and remove excess food repeatedly photographed when calculating the nutritional content of food.

At step 2002, after the patient takes a image of the food, if the food image recognition model is set on the smart device, the food image can be directly transmitted to a food image recognition application (hereinafter referred to as APP) in which the food image recognition model is located, the APP can be downloaded onto the smart device by the patient or his care giver, or the APP is a subroutine of a closed-loop artificial pancreas program in which the identification of the food image can be done.

At step 2003, after the APP finishes recognition on the food image, nutrient content (mainly comprising carbohydrate, fat and protein content) in the food image is available, presenting to a patient through an interactive interface, the presentation including the identified food species, weight and corresponding nutrient content and total nutrient content, the interactive interface can also prompt the patient whether to confirm the presentation, food species, weight and corresponding nutrient content and total nutrient content as derived from patient confirmed food image recognition model, ready to be used to calculate an insulin infusion amount, if the patient considers that there is a significant deviation in the type, weight and corresponding nutrient content and total nutrient content of the food obtained by the food image recognition model, the patient can choose to re-recognize the food image, or to re-photograph the food until the recognition result of the food image recognition model is confirmed by the patient.

At step 2004, the APP transmits the aggregate nutrient content into the closed-loop artificial pancreas, based on the aggregate nutrient content, invoking an insulin algorithm completes the calculation of an insulin infusion amount and completes the infusion, the postprandial insulin infusion amount including a basal amount and a bolus amount, while the system also simulates a simulated blood glucose profile *l₁* of the patient for a period of time after eating based on the calculated insulin infusion amount. In the present step, assuming that the insulin algorithm is stored in a closed-loop artificial pancreas, the insulin algorithm can also be stored in the APP, after the APP finishes recognition of the food image, the calculation of the postprandial insulin infusion amount can be done locally directly, and then the postprandial insulin infusion amount data is transmitted to the closed-loop artificial pancreas, where the infusion is done. In the present step, the called insulin algorithm can be an insulin algorithm stored in an application or a closed-loop artificial pancreas, can also be an algorithm located in the cluster of narrow insulin algorithms, calling an algorithm in the cluster of narrow insulin algorithm requires the patient to first determine an identifiable feature of himself, and to retrieve an insulin algorithm suitable for himself based on the identifiable feature, the more identifiable features, the more suitable the retrieved insulin algorithm is for the patient himself, and thus the more accurate the calculated postprandial insulin infusion amount.

At step 2005, while starting the postprandial insulin infusion, the detection mechanism 100 detects and records postprandial actual blood glucose data of the patient to form an actual blood glucose profile 12 of the patient for a period of time, e.g., 2-5 hours, which coincides with the duration of the simulated blood glucose profile *l₁*.

At step 2006, after the detecting module 100 records blood glucose data for a preset time, the system compares the data of the simulated blood glucose profile *l₁* and the data of the actual blood glucose profile *l₂,* modifying insulin algorithm parameters according to the comparison result, gradually bringing the simulated blood glucose profile *l₁* into agreement with the actual blood glucose profile *l₂,* the parameter corrected narrow insulin algorithm will be more suitable for this patient, the parameter corrected narrow insulin algorithm can therefore be referred to as narrow insulin algorithm, meanwhile, on the one hand, the system returns the comparison result to the APP, the APP can make revisions to the food image recognition model parameters to optimize the food image recognition model, the insulin algorithm and the food image recognition model after parameter revisions will be used for food image recognition and insulin calculation at the next meal. On the other hand, after patient consent, the parameter corrected insulin algorithms can be grouped into cluster of narrow insulin algorithm and sorted into corresponding groups according to the patient's identifiable features for invocation by the patient or other patients with the same identifiable features before a meal.

In some embodiments of the invention, due to the limited computational power and storage capacity of smart devices or closed-loop artificial pancreas, the cloud server 104 can be plugged into a closed-loop artificial pancreas system in view of the fact that sophisticated image recognition and insulin infusion amount calculations cannot be supported, enough insulin narrow algorithms cannot be stored, and a sufficiently rich assemble of food nutrients cannot be stored. The cloud server 104 can communicate with the smart device or closed-loop artificial pancreas via wired or wireless means to enable data interaction. Upon accessing the cloud server 104, the cloud server 104 can provide powerful computational and storage capacity support for closed-loop artificial pancreas and smart devices, an image recognition model, an insulin narrow algorithm cluster, or a food nutrition library can be stored in the cloud server 104, and completing the identification of the food image or the calculation of the insulin infusion amount in the cloud server 104, or the comparison of the simulated blood glucose data and the actual blood glucose data is done, and even the correction of the parameters of the insulin algorithm or the image recognition model is done, so that the food recognition during meals can be done without using the strong-performing closed-loop artificial pancreas and the smart device, the calculation of the insulin infusion amount, and the use cost is saved for the patient.

After accessing the cloud server 104, the method of using the closed-loop artificial pancreas system will be corrected, with reference to FIG. 11c, as follows:
In step 3001, a patient takes a food image for a food using a smart device, and the food image is recognized by a food image recognition model.

In step 3002, the food image recognition model is set in the cloud server 104, the patient, after photographing the food image, uploads the food image to the cloud server 104 through the smart device, and completes the food image recognition.

At step 3003, the cloud server 104 returns the identified food type, weight and corresponding nutrient content and aggregate nutrient content data to the smart device, and presenting to the patient through the interactive interface, food species, weight and corresponding nutrient content and total nutrient content as derived from patient confirmed food image recognition model, ready for use in calculating a postprandial insulin infusion amount, if the patient considers that there is a significant deviation in the type, weight and corresponding nutrient content and total nutrient content of the food obtained by the food image recognition model, the patient can choose to re-recognize the food image, or to re-photograph the food until the recognition result of the food image recognition model is confirmed by the patient.

At step 3004, patient calls insulin algorithm, or retrieving the same insulin algorithm with self-identifiable features from the cluster of insulin algorithms in narrow sense, based on the determined insulin algorithm and nutrient content, calculating, by the cloud server 104, a postprandial insulin infusion amount of the patient, and simulating a postprandial blood glucose change of the patient to obtain simulated blood glucose data, forming a simulated blood glucose profile *l₁,* the cloud server 104 transmits the postprandial insulin infusion amount data to the closed-loop artificial pancreas and completes infusion, the postprandial insulin infusion amount includes a basal amount and a bolus amount.

At step 3005, while starting insulin infusion, the detection mechanism 100 detects, records actual blood glucose data of the patient, forms an actual blood glucose profile 12 for a period of time, e.g., 2-5 hours, which coincides with the duration of the simulated blood glucose profile *l₁*, and transmits the actual blood glucose data for the period of time to the cloud server 104.

At step 3006, the cloud server 104 compares the simulated blood glucose profile *l₁* data and the actual blood glucose profile *l₂* data, the parameters of the insulin algorithm is corrected according to the comparison result so that the simulated blood glucose profile *l₁* and the actual blood glucose profile *l₂* gradually tend to coincide, and the parameter corrected insulin algorithm will be more suitable for the patient, so that the parameter corrected insulin algorithm can be referred to as the insulin algorithm of the narrow sense. Meanwhile, the cloud server 104 uses the comparison result and the correction result to the insulin algorithm parameters for adjusting the food image recognition model parameters, to optimize the food image recognition model, on the one hand, the narrow insulin algorithm and food image recognition model will be used for food image recognition and insulin calculation at the next meal, alternatively, if agreed upon by the patient, the insulin sensations algorithm can be grouped into clusters of narrow insulin algorithms, sorted into corresponding groups according to the identifiable features of the patient, and replaced with the insulin algorithm prior to parameter correction for invocation by the patient or other patients with the same identifiable features prior to the meal.

In an embodiment of the invention, the calculation of the insulin infusion amount can be performed in the closed-loop artificial pancreas, or the smart device, or the cloud server 104, and the implementation thereof will not be described in detail.

In the embodiment of the invention, the identification of the food image can be performed in the closed-loop artificial pancreas, or the smart device, or the cloud server 104, and the implementation thereof will not be described in detail.

In an embodiment of the invention, the simulation calculation of the simulated blood glucose profile *l₁* can be performed in the closed-loop artificial pancreas, or in the smart device, or in the cloud server 104, and the implementation thereof will not be described in detail.

Referring to FIG. 12, another closed-loop infusion method based on food image recognition is illustrated in accordance with an embodiment of the invention. The insulin algorithm can be integrated in the food image recognition model, and after the food image recognition model recognizes the type and weight of food, the postprandial insulin infusion amount can be determined directly by the insulin algorithm without recognizing the nutritional content of food, as described below. The normalized model of the food image recognition model and the insulin algorithm is referred to as a big model in the invention.

In embodiments of the invention, the insulin algorithm can be divided into logical operations and look-up tables, as described below.

In an embodiment of the invention, the big model can be stored in the smart device, the closed-loop artificial pancreas system, or the cloud server 104, and after receiving the food image input, the postprandial insulin infusion amount can be directly output and the postprandial insulin infusion is completed by the closed-loop artificial pancreas.

Taking the big model stored in the intelligent device as an example, in the embodiment of the invention, in the step 4001, the big model still needs to use the imaging mechanism 103 such as the intelligent device to acquire the food image, and the step of acquiring the food image is consistent with the foregoing and will not be repeated here.

In step 4002, the food image is transmitted to a food image recognition application, and a big model in the APP recognizes the type and weight of food in the food image.

At step 4003, after the big model recognizes the type and weight of food in the food image, the patient is presented and prompted whether to confirm or not through the interactive interface of the intelligent device, as the patient confirms the type and weight of food presented by the interactive interface, the postprandial insulin infusion amount can be output through the preset insulin algorithm. If the patient considers that there is a significant deviation in the type and weight of the identified food, the patient can choose to re-identify the food image, or to re-photograph the food and perform the identification until the identification result of the big model is confirmed by the patient.

At step 4004, after the patient confirms the type and weight of the food, based on the type and weight of the food, the big model derives the postprandial insulin infusion amount of the patient through a preset insulin algorithm. The preset insulin algorithm can be one or more of neural network, PID, MPC, etc. logic operations, the postprandial insulin infusion amount can be calculated according to the type and weight of foods, and the big model calculates the postprandial insulin infusion amount for each food separately after identifying the food image, and then adds the postprandial insulin infusion amounts for all foods to a total postprandial insulin infusion amount. For example, a big model versus a patient-provided food image, recognizing 500g for potatoes, 250g for potatoes and 250g for beef, and calculating by a preset insulin algorithm that the postprandial insulin infusion amount of 500g for potatoes is I1, 250g for potatoes is I2, and 250g for beef is I3, then the total postprandial insulin infusion amount of the patient is I1+I2+I3, which is described only schematically.

The preset insulin algorithm can also be a look-up table, retrieving a postprandial insulin infusion amount according to the type and weight of food based on a look-up table, postprandial insulin infusion amounts for each food and its respective weight are stored in the big model in the form of a look-up table, as shown in Table 3.1, for example, a big model versus a patient-provided food image, recognizing 300g for potatoes, 200g for notes and 200g for beef, and by searching the look-up table, it is found that the postprandial insulin infusion for 300g potatoes is 14, that the postprandial insulin infusion for 200g for notes is 15, and that the postprandial insulin infusion for 200g for beef is 16, then the total postprandial insulin infusion for this patient is 14+15+16, as just described schematically.

**Table 3.1 Food-Insulin Lookup Table**

| Type of Food | Weight of Food/g | Insulin Infusion Amount /U |
|---|---|---|
| potato | 100 | - |
| | 200 | - |
| | 300 | I₄ |
| | 400 | - |
| | ... | - |
| beef | 100 | - |
| | 200 | I₆ |
| | 300 | - |
| | 400 | - |
| | ... | - |
| noodle | 100 | - |
| | 200 | I₅ |
| | 300 | - |
| | 400 | - |
| | ... | - |
| ... | ... | ... |

Table 3.1 is an expression of a look-up table, comprising at least a column "food type", a column "food weight" and a column "insulin infusion amount", in some more detailed look-up tables, some additional food information such as "place of origin of food" column, "cooking mode" column, "storage time of food" column, etc. can be included, and after taking the image of food, the patient can select the additional food-related information at the interactive interface of the smart device to help the food image recognition model to better recognize the image of food. Food additional information is key information affecting food nutrient content, such information can be difficult to recognize by the food image recognition model, which can have a certain influence on the quantitative analysis of nutrients in food, and therefore information corresponding to food needs to be input or selected by the patient before the food image is recognized as additional information before the food image is recognized.

In a big model, either by means of a logical operation or a look-up table to derive the postprandial insulin infusion amount, initially, commonly used empirical parameters were employed, the same type of food can be due to meal, patient digestion level, cooking pattern, etc. The same type and weight of food eventually affect blood glucose differently from patient to patient, and the insulin algorithm cannot take into account the various influences in an exhaustive manner, so that the insulin algorithm can be corrected.

After determining the postprandial insulin infusion amount for the patient, the big model will simulate the patient's postprandial blood glucose data based on the postprandial insulin infusion amount to form a simulated blood glucose profile *l₃,* the simulation lasting a period of time, e.g., 2-5 hours.

At step 4005, the APP transmits the patient's postprandial insulin infusion amount data to the closed-loop artificial pancreas, and the system starts the postprandial insulin infusion, and at the same time, the system records the actual blood glucose data detected by the detection mechanism 100 to form an actual blood glucose profile *l₄*, and the duration of the actual blood glucose profile *l₄* coincides with the duration of the simulated blood glucose profile *l₃.*

At step 4006, the system transmits actual blood glucose data to the smart device, the big model compares the difference between the simulated blood glucose profile *l₃* data and the actual blood glucose profile *l*₄ data, and corrects the simulated blood glucose profile until it is consistent with the actual blood glucose profile, and the comparison result is used to correct the insulin logic operation parameters preset in the big model or the look-up table data, and can even be used to correct the image recognition model parameters.

In an embodiment of the invention, insulin logic operation parameters or look-up table data after each revision, it will gradually adapt to the eating habits of the patient, so the correction process of the parameters of the insulin algorithm in the present scheme is the learning process, the insulin algorithm is more and more adapted to the physiological characteristics and the living habits of the patient during the learning process, whereby the calculated postprandial insulin infusion amount will be more accurate, helping the patient's diabetes treatment.

In an embodiment of the invention, the look-up table is corrected by modifying the data in the "Insulin Infusion Amount" column, adding, modifying or deleting the data in the "Category", "Weight" columns, and the corrected look-up table can still be assembled and sorted into groups according to patient identifiable features for other patients to call. As previously described, the look-up table after correction can be referred to as a narrow look-up table to distinguish it from the look-up table before correction.

In some embodiments of the invention, the food image recognition model and the insulin algorithm can be separate and stored in separate modules. For example, the food image recognition model is stored in the smart device, the insulin algorithm is stored in one of the detection mechanism 100, the program mechanism 101 or the infusion mechanism 102 of the closed-loop artificial pancreas system, when the smart device completes the food image recognition, the food type and weight data are transmitted into the closed-loop artificial pancreas, the calculation of the insulin amount is done by the system or querying and the insulin infusion is done. For another example, the food image recognition model is stored in the cloud server and the insulin algorithm is stored in the smart device. As another example, the food image recognition model is stored in a cloud server and the insulin algorithm is stored in one of the detection mechanism 100, the program mechanism 101, or the infusion mechanism 102 of the closed-loop artificial pancreas system.

In an embodiment of the invention, the above partial steps are exchangeable in order, e.g. For the calculation step of simulated blood glucose data, either before the step of acquiring actual blood glucose data or after the step of acquiring actual blood glucose data can be provided, which does not affect the postprandial insulin calculation for the patient, the infusion, nor the comparison of simulated blood glucose data to actual blood glucose data to correct narrow insulin algorithm parameters.

It will be appreciated by those skilled in the art that the pre-step exchange and the post-step exchange schemes are intended to be included within the scope of the invention without affecting the exchange of steps performed by the scheme.

It will be understood by those skilled in the art that regardless of the normalization or separation of the food image recognition model and the insulin algorithm, the storage position is changed and should be included in the scope of the invention.

In summary, the invention discloses a method and system for postprandial insulin infusion based on identifiable feature of diabetic, acquiring a food image by an imaging mechanism, and analyzing the food image by the food image recognition model to obtain food data, retrieving a narrow insulin algorithm adapted to the patient's own identifiable features from the cluster of narrow insulin algorithms according to the identifiable features of the patient, for calculation of insulin infusion amount during meals, compared to the preset insulin algorithm, the narrow insulin algorithm is more applicable to the physiological characteristics and living habit of the patient, the calculated postprandial insulin infusion amount is more accurate, and is beneficial for the treatment of diabetic.

While the invention has been described in detail with reference to the specific embodiments of the invention, it should be understood that it will be appreciated by those skilled in the art that the above embodiments can be corrected without departing from the scope and spirit of the invention. The scope of the invention is defined by the appended claims.

## Claims

1. A closed-loop artificial pancreas system comprising:
an imaging mechanism for acquiring food images on food before a meal;
a food image recognition model for recognizing a food image to acquire food data, the food data including at least type and weight of food;
a cluster of narrow insulin algorithms assembled by narrow insulin algorithms, in the cluster of narrow insulin algorithm, the narrow insulin algorithms are classified into groups with identifiable features, with common identifiable features within the groups, the patient retrieves the narrow insulin algorithm based on the identifiable features, and determines a postprandial insulin infusion amount of the patient using the narrow insulin algorithm based on the food data; and
a closed-loop artificial pancreas for completing the infusion of postprandial insulin and detecting actual blood glucose of the patient, obtaining actual blood glucose data.

2. The closed-loop artificial pancreas system of claim 1, wherein the groups are independent of each other.

3. The closed-loop artificial pancreas system of claim 1, wherein the two groups having at least one same identifiable feature are subsets of each other.

4. The closed-loop artificial pancreas system of claim 1, further comprising a cloud server, the cloud server establishing communication with the imaging mechanism or the closed-loop artificial pancreas.

5. The closed-loop artificial pancreas system of claim 1, further comprising a food nutrition library, the food data further comprising the content of nutrients of the food, the food nutrition library being retrieved based on the type and weight of the food to determine the content of nutrients of the food.

6. The closed-loop artificial pancreas system of claim 5, wherein the narrow insulin algorithm determines the postprandial insulin infusion amount based on the content of nutrients.

7. The closed-loop artificial pancreas system of claim 5, wherein the cluster of narrow insulin algorithms is located in the imaging mechanism, the closed-loop artificial pancreas or the cloud server.

8. The closed-loop artificial pancreas system of claim 5, wherein the image recognition model is located in the imaging mechanism, the closed-loop artificial pancreas or the cloud server.

9. The closed-loop artificial pancreas system of claim 5, wherein the food nutrition library is located in the imaging mechanism, the closed-loop artificial pancreas or the cloud server.

10. The closed-loop artificial pancreas system of claim 1, wherein the identifiable features are associated with at least a physiological state or living habit of the patient, and identifiable features may be added, modified or deleted.

11. The closed-loop artificial pancreas system of claim 1, wherein the imaging mechanism is a closed-loop artificial pancreas independent image acquisition device.

12. The closed-loop artificial pancreas system of claim 1, wherein the imaging mechanism is a sub-module of the closed-loop artificial pancreas.

13. The closed-loop artificial pancreas system of claim 1, wherein, the narrow insulin algorithm is also used to simulate the postprandial blood glucose of the patient, based on the postprandial insulin infusion amount, resulting in simulated blood glucose data.

14. The closed-loop artificial pancreas system of claim 13, wherein the actual blood glucose data and the simulated blood glucose data are compared, and the parameters of the narrow insulin algorithm and/or the image recognition model are corrected according to the comparison result.

15. A closed-loop insulin infusion method, comprising providing: an imaging mechanism, a food image recognition model, a cluster of narrow insulin algorithm and a closed-loop artificial pancreas, comprising the steps of:
I. Before a meal, acquiring a food image for the food using an imaging mechanism;
II. Recognizing the food image by the food image recognition model, acquiring food data related to the food;
III. Retrieving a narrow insulin algorithm according to the identifiable characteristics of the patient from the cluster of insulin algorithms, determining a postprandial insulin infusion amount of the patient by the insulin algorithm based on the food data;
IV. Completing the postprandial insulin infusion by the closed-loop artificial pancreas and detecting, recording actual blood glucose data.

16. The closed-loop insulin infusion method of claim 15, wherein, in the step II, further comprising a step of confirming the food data by the patient, and if the food data is confirmed by the patient, proceeding to the step III, and vice versa, returning to the step I, or re-recognizing the food image by the food image recognition model.

17. The closed-loop insulin infusion method of claim 15, wherein, in the step II, the food data acquired include at least the type and weight of the food.

18. The closed-loop insulin infusion method of claim 17, wherein, further comprising providing a food nutrient library, retrieving the food nutrient library to determine the nutritional content of the food based on the type and weight of the food.

19. The closed-loop insulin infusion method of claim 18, wherein, in the step III, the postprandial insulin infusion amount is determined by the narrow insulin algorithm based on the nutritional content of the food.

20. The closed-loop insulin infusion method of claim 15, wherein, further comprising providing a cloud server in which the image recognition model, the cluster of narrow insulin algorithms or the food nutrition library is stored.

21. The closed-loop insulin infusion method of claim 20, wherein, in the step II, further comprising uploading the food image to the cloud server, and completing food image recognition in the cloud server.

22. The closed-loop insulin infusion method of claim 20, wherein, in the step III, further comprising uploading the food data to the cloud server, and completing the calculation of the postprandial insulin infusion amount in the cloud server.

23. The closed-loop insulin infusion method of claim 17, wherein, in the step III, further comprising determining the postprandial insulin infusion amount by the narrow insulin algorithm based on the type and weight of the food.

24. The closed-loop insulin infusion method of claim 23, wherein, further comprising simulating a postprandial blood glucose of the patient based on the postprandial insulin infusion amount, and obtaining the simulated blood glucose data.

25. The closed-loop insulin infusion method of claim 24, wherein, further comprising step:
V . Comparing actual blood glucose data and simulated blood glucose data, the parameters of the narrow insulin algorithm are corrected based on the comparison results.

26. The closed-loop insulin infusion method of claim 25, wherein, the actual blood glucose data coincides in time with the simulated blood glucose data.

27. The closed-loop insulin infusion method of claim 25, wherein, the narrow insulin algorithm with parameters corrected is assembled into the cluster of narrow insulin algorithm and replaces the narrow insulin algorithm before parameter correction.
